# EUROPEAN PATENT APPLICATION

(11) **EP 4 653 423 A1**
(43) Date of publication of application: **26.11.2025**
(21) Application number: 24778046.3
(22) Date of filing: 27.03.2024
(51) Int. Cl.: C07D 231/20, C07D 231/12, A01N 43/56

(54) **PYRAZOL-5-ETHER COMPOUND, AND PREPARATION METHOD THEREFOR AND USE THEREOF**

(30) Priority: 27.03.2023 CN 202310307179; 26.03.2024 CN 202410349968
(71) Applicant: CAC Nantong Chemical Co., Ltd., 226407 Nantong City Jiangsu (CN)
(72) Inventor: LV, Liang, Nantong, Jiangsu 226407 (CN); LI, Hongwei, Nantong, Jiangsu 226407 (CN); CAO, Xiaofeng, Nantong, Jiangsu 226407 (CN); WANG, Jine, Nantong, Jiangsu 226407 (CN); DENG, Denghui, Nantong, Jiangsu 226407 (CN); DU, Yonglei, Nantong, Jiangsu 226407 (CN); CHEN, Chen, Nantong, Jiangsu 226407 (CN); WANG, Ding, Nantong, Jiangsu 226407 (CN)
(74) Representative: Grünecker Patent- und Rechtsanwälte PartG mbB
(86) International application number: PCT/CN2024/083993
(87) International publication number: WO 2024/199260

(57) **Abstract**

A pyrazol-5-ether compound having a structure represented by Formula I, which has a significant effect on preventing and controlling diseases in agriculture and forestry, especially on wheat powdery mildew, cucumber powdery mildew and soybean rust, and has wide application prospects.

## Description

### TECHNICAL FIELD

The present application belongs to the field of agricultural fungicides and relates to a pyrazol-5-ether compound, a preparation method therefor and the use thereof.

### BACKGROUND

Plant diseases severely impact plant growth and lead to reduced yields in crops, ornamental plants, pastures, and forestry, thereby causing economic losses. With the long-term, extensive use of existing fungicides, pathogens develop resistance to commercially available fungicides. Thus, new and improved fungicidal compounds that are more effective, more cost-efficient, less toxic and environmentally safer and/or possess different modes of action are needed to be developed.

In the existing art, several pyrazole ether compounds have been reported. There are pyrazol-5-ether compounds. For example, CN101631460A discloses KC1 and KC2 (i.e., compounds 596 and 599 of CN101631460A, respectively). These disclosed compounds have herbicidal activities, but no specific biological testing experiment data on fungicidal activities of pyrazol-5-ether compounds have been reported. There are also pyrazol-3-ether compounds. For example, CN116003322A discloses KC3 (i.e., compound 22 of CN116003322A), and the disclosed compound has fungicidal activities against cucumber powdery mildew.

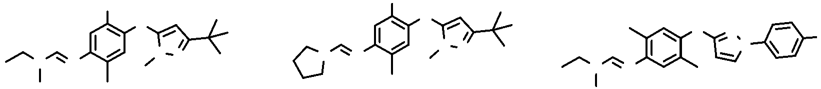

In the art, it is still desirable to develop more efficient fungicides to meet the demands of agriculture and forestry industries.

### SUMMARY

The present application provides a pyrazol-5-ether compound, a preparation method therefor and the use thereof.

In a first aspect, the present application provides a pyrazol-5-ether compound. The pyrazol-5-ether compound has a structure represented by Formula I: 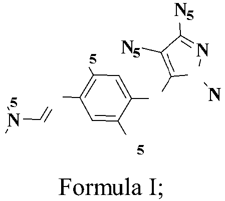 wherein
R₁ is selected from C1-C6 alkyl, C3-C6 cycloalkyl or phenyl group, which is substituted with at least one R₈;
R₂ is selected from C1-C6 haloalkyl, C3-C6 cycloalkyl, methyl, cyano or phenyl group, the phenyl group is substituted with at least one R₈;
R₃ is selected from hydrogen, halogen, cyano, C1-C6 alkyl or C1-C6 haloalkyl group;
R₄ is selected from halogen, C1-C6 alkyl, C1-C6 alkoxy, C1-C6 haloalkyl or C1-C6 haloalkoxy group;
R₅ is selected from hydrogen, halogen, C1-C6 alkyl, C1-C6 alkoxy or C1-C6 haloalkyl group;
R₆ is selected from C1-C8 alkyl, C3-C6 cycloalkyl or C1-C6 haloalkyl group, which is substituted with at least one R₈;
R₇ is selected from hydrogen or C1-C6 alkyl group; or R₆ and R₇, together with the nitrogen atom to which they are attached, form a 3- to 7-membered saturated cyclic group, the cyclic group may optionally contain an oxygen atom, a nitrogen atom or a sulfur atom, and the cyclic group may be further substituted with identical or different R₈; and
R₈ is selected from hydrogen, halogen, cyano, nitro, hydroxyl, amino, C1-C6 alkyl, C1-C6 cycloalkyl, C1-C6 haloalkyl, C1-C6 alkoxy, C1-C6 haloalkoxy, amino substituted with C1-C6 alkyl, C1-C6 alkylcarbonylamino, C1-C6 haloalkylcarbonylamino, C1-C6 alkylsulfonylamino, *N,N-di(C1-C6* alkyl)sulfonylamino, C1-C6 haloalkylsulfonylamino, heterocycloacylamino, C1-C6 alkylcarbonyl, C1-C6 alkyloxycarbonyl, cyano-substituted C1-C6 alkyl, C1-C6 alkylsulfinyl, C1-C6 alkylsulfonyl, C1-C6 haloalkylsulfinyl, C1-C6 haloalkylsulfonyl, substituted phenyl, a heterocyclic ring or heterocyclic-substituted C1-C6 alkyl group.

In the present application, as a preferred technical solution, in Formula I,
R₁ is selected from methyl, ethyl, n-propyl, isopropyl, n-butyl, isobutyl, tert-butylcyclopropyl, cyclobutyl, cyclopentyl, cyclohexyl or phenyl group, which is substituted with at least one R₈;
R₂ is selected from methyl, cyclopropyl, difluoromethyl, trifluoromethyl, cyano or phenyl group, the phenyl group is substituted with at least one R₈;
R₃ is selected from hydrogen, fluorine, chlorine, bromine, iodine, cyano, methyl, difluoromethyl or trifluoromethyl group;
R₄ is selected from methyl, difluoromethyl, trifluoromethyl, methoxy, difluoromethoxy, trifluoromethoxy, fluorine, chlorine, bromine or iodine group;
R₅ is selected from hydrogen, methyl, methoxy, difluoromethyl, trifluoromethyl, fluorine, chlorine, bromine or iodine group;
R₆ is selected from methyl, ethyl, n-propyl, isopropyl, 1-methylpropyl, 1-ethylpropyl, 2-methylpropyl, 3-methylpropyl, 1,1-dimethylpropyl, 2,2-dimethylpropyl, n-butyl, *tert-butyl,* 3-methylbutyl, 1,1-dimethyl-3,3-dimethylbutyl, n-pentyl, 4-methyl-2-pentyl, 1-methylpentyl, 2-methylpentyl, 3-methylpentyl, 4-methylpentyl, n-hexyl, 2-ethylhexyl, cyclopropyl, cyclobutyl, cyclopentyl, cyclohexyl or cyclopropylmethyl group;
R₇ is selected from hydrogen, methyl, ethyl, n-propyl, isopropyl, n-butyl, isobutyl, *tert-butyl,* n-pentyl, 2-pentyl, neopentyl, isopentyl, 4-methyl-2-pentyl or n-hexyl group; or R₆ and R₇, together with the nitrogen atom to which they are attached, form tetrahydropyrrole, isoxazolidine, piperidine, azacyclooctane, piperazine or morpholine, and the tetrahydropyrrole, isoxazolidine, piperidine, azacyclooctane, piperazine or morpholine may be substituted with identical or different R₈;
R₈ is selected from hydrogen, fluorine, chlorine, bromine, iodine, cyano, nitro, hydroxyl, amino, methyl, ethyl, n-propyl, isopropyl, isobutyl, n-butyl, *tert-butyl,* cyanomethyl, difluoromethyl, trifluoromethyl, pentafluoroethyl, heptafluoroisopropyl, methoxy, difluoromethoxy, trifluoromethoxy, acetyl, tert-butoxycarbonyl, amino, methylamino, acetylamino, trifluoroacetylamino, methanesulfonamido, trifluoromethanesulfonamido, N,N-dimethylaminosulfonamido, morpholine-4-formamido, pyrazolyl, imidazolyl, 1,2,4-triazolyl, 1,2,3-triazolyl, 1,3,4-triazolyl, pyrazolinyl, imidazolinyl, 1,2,4-triazolidinyl, pyridyl, pyrimidinyl, pyridazinyl, pyrazinyl, oxadiazolyl, thiadiazolyl, isoxazolidinyl or isoxazolinyl group.

As a preferred technical solution of the present application, the pyrazol-5-ether compound is any one of the compounds having General Formula I in Table 1:

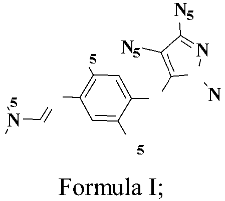

**Table 1**

| Comp. | R₁ | R₂ | R₃ | R₄ | R₅ | 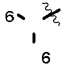 | Appearance (melting point) |
|---|---|---|---|---|---|---|---|
| 1 | methyl | trifluoromethyl | hydrogen | methyl | methyl | 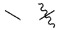 | |
| 2 | methyl | trifluoromethyl | hydrogen | methyl | methyl | 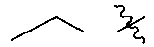 | Yellow solid (108.8-111.2) |
| 3 | methyl | trifluoromethyl | hydrogen | methyl | methyl | 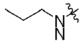 | Yellow solid (90.5-94.6) |
| 4 | methyl | trifluoromethyl | hydrogen | methyl | methyl | 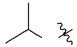 | |
| 5 | methyl | trifluoromethyl | hydrogen | methyl | methyl | 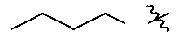 | |
| 6 | methyl | trifluoromethyl | hydrogen | methyl | methyl | 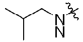 | |
| 7 | methyl | trifluoromethyl | hydrogen | methyl | methyl | 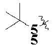 | |
| 8 | methyl | trifluoromethyl | hydrogen | methyl | methyl | 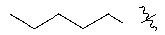 | |
| 9 | methyl | trifluoromethyl | hydrogen | methyl | methyl | 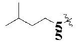 | |
| 10 | methyl | trifluoromethyl | hydrogen | methyl | methyl | 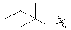 | |
| 11 | methyl | trifluoromethyl | hydrogen | methyl | methyl | 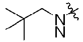 | |
| 12 | methyl | trifluoromethyl | hydrogen | methyl | methyl | 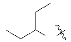 | |
| 13 | methyl | trifluoromethyl | hydrogen | methyl | methyl | 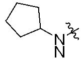 | |
| 14 | methyl | trifluoromethyl | hydrogen | methyl | methyl | 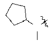 | |
| 15 | methyl | trifluoromethyl | hydrogen | methyl | methyl | 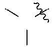 | Yellow liquid |
| 16 | methyl | trifluoromethyl | hydrogen | methyl | methyl | 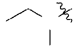 | Yellow liquid |
| 17 | methyl | trifluoromethyl | hydrogen | methyl | methyl | 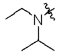 | |
| 18 | methyl | trifluoromethyl | hydrogen | methyl | methyl | 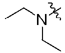 | Yellow liquid |
| 19 | methyl | trifluoromethyl | hydrogen | methyl | methyl | 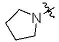 | Yellow liquid |
| 20 | methyl | trifluoromethyl | hydrogen | methyl | methyl | 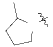 | |
| 21 | methyl | trifluoromethyl | hydrogen | methyl | methyl | 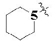 | |
| 22 | methyl | trifluoromethyl | hydrogen | methyl | methyl | 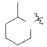 | |
| 23 | methyl | trifluoromethyl | hydrogen | methyl | methyl | 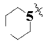 | |
| 24 | methyl | trifluoromethyl | hydrogen | methyl | methyl | 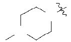 | |
| 25 | methyl | trifluoromethyl | chlorine | methyl | methyl | 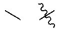 | |
| 26 | methyl | trifluoromethyl | chlorine | methyl | methyl | 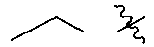 | |
| 27 | methyl | trifluoromethyl | chlorine | methyl | methyl | 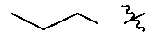 | |
| 28 | methyl | trifluoromethyl | chlorine | methyl | methyl | 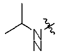 | |
| 29 | methyl | trifluoromethyl | chlorine | methyl | methyl | 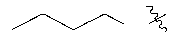 | |
| 30 | methyl | trifluoromethyl | chlorine | methyl | methyl | 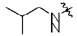 | |
| 31 | methyl | trifluoromethyl | chlorine | methyl | methyl | 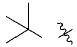 | |
| 32 | methyl | trifluoromethyl | chlorine | methyl | methyl | 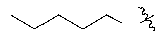 | |
| 33 | methyl | trifluoromethyl | chlorine | methyl | methyl | 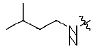 | |
| 34 | methyl | trifluoromethyl | chlorine | methyl | methyl | 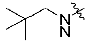 | |
| 35 | methyl | trifluoromethyl | chlorine | methyl | methyl | 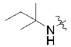 | |
| 36 | methyl | trifluoromethyl | chlorine | methyl | methyl | 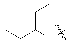 | |
| 37 | methyl | trifluoromethyl | chlorine | methyl | methyl | 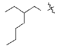 | |
| 38 | methyl | trifluoromethyl | chlorine | methyl | methyl | 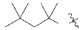 | |
| 39 | methyl | trifluoromethyl | chlorine | methyl | methyl | 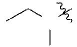 | |
| 40 | methyl | trifluoromethyl | chlorine | methyl | methyl | 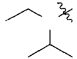 | |
| 41 | methyl | trifluoromethyl | chlorine | methyl | methyl | 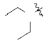 | |
| 42 | methyl | trifluoromethyl | chlorine | methyl | methyl | 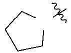 | |
| 43 | methyl | trifluoromethyl | chlorine | methyl | methyl | 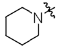 | |
| 44 | methyl | trifluoromethyl | chlorine | methyl | methyl | 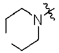 | |
| 45 | methyl | trifluoromethyl | bromine | methyl | methyl | 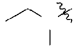 | |
| 46 | methyl | trifluoromethyl | fluorine | methyl | methyl | 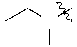 | |
| 47 | methyl | trifluoromethyl | iodine | methyl | methyl | 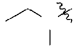 | |
| 48 | methyl | trifluoromethyl | cyano | methyl | methyl | 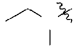 | |
| 49 | methyl | trifluoromethyl | methyl | methyl | methyl | 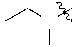 | Yellow oily liquid |
| 50 | methyl | trifluoromethyl | hydrogen | chlorine | methyl | 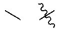 | |
| 51 | methyl | trifluoromethyl | hydrogen | chlorine | methyl | 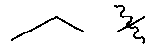 | |
| 52 | methyl | trifluoromethyl | hydrogen | chlorine | methyl | 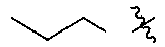 | |
| 53 | methyl | trifluoromethyl | hydrogen | chlorine | methyl | 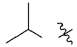 | |
| 54 | methyl | trifluoromethyl | hydrogen | chlorine | methyl | 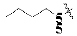 | |
| 55 | methyl | trifluoromethyl | hydrogen | chlorine | methyl | 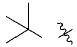 | |
| 56 | methyl | trifluoromethyl | hydrogen | chlorine | methyl | 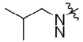 | |
| 57 | methyl | trifluoromethyl | hydrogen | chlorine | methyl | 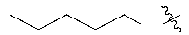 | |
| 58 | methyl | trifluoromethyl | hydrogen | chlorine | methyl | 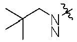 | |
| 59 | methyl | trifluoromethyl | hydrogen | chlorine | methyl | 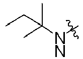 | |
| 60 | methyl | trifluoromethyl | hydrogen | chlorine | methyl | 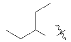 | |
| 61 | methyl | trifluoromethyl | hydrogen | chlorine | methyl | 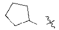 | |
| 62 | methyl | trifluoromethyl | hydrogen | chlorine | methyl | 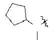 | |
| 63 | methyl | trifluoromethyl | hydrogen | chlorine | methyl | 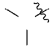 | Yellow oily liquid |
| 64 | methyl | trifluoromethyl | hydrogen | chlorine | methyl | 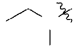 | Yellow oily liquid |
| 65 | methyl | trifluoromethyl | hydrogen | chlorine | methyl | 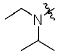 | |
| 66 | methyl | trifluoromethyl | hydrogen | chlorine | methyl | 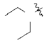 | Yellow oily liquid |
| 67 | methyl | trifluoromethyl | hydrogen | chlorine | methyl | 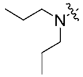 | Colorless oily liquid |
| 68 | methyl | trifluoromethyl | hydrogen | chlorine | methyl | 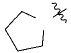 | Yellow oily liquid |
| 69 | methyl | trifluoromethyl | hydrogen | chlorine | methyl | 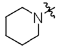 | Yellow oily liquid |
| 70 | methyl | trifluoromethyl | hydrogen | methyl | chlorine | 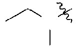 | Yellow oily liquid |
| 71 | methyl | trifluoromethyl | hydrogen | methyl | chlorine | 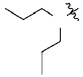 | Yellow oily liquid |
| 72 | methyl | trifluoromethyl | hydrogen | methyl | chlorine | 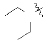 | Yellow oily liquid |
| 73 | methyl | trifluoromethyl | hydrogen | methyl | chlorine | 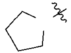 | Yellow oily liquid |
| 74 | methyl | trifluoromethyl | hydrogen | methyl | chlorine | 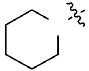 | Yellow oily liquid |
| 75 | methyl | trifluoromethyl | hydrogen | methyl | chlorine | 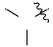 | Yellow oily liquid |
| 76 | methyl | trifluoromethyl | hydrogen | chlorine | methoxy | 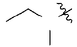 | Yellow liquid |
| 77 | methyl | trifluoromethyl | hydrogen | chlorine | methoxy | 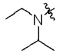 | Yellow solid (83.5-86.1) |
| 78 | methyl | trifluoromethyl | hydrogen | methyl | methyl | 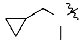 | |
| 79 | methyl | difluoromethyl | hydrogen | methyl | methyl | 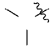 | Light brown oil |
| 80 | methyl | difluoromethyl | hydrogen | methyl | methyl | 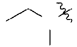 | Light brown oil |
| 81 | methyl | difluoromethyl | hydrogen | methyl | methyl | 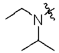 | |
| 82 | methyl | difluoromethyl | hydrogen | methyl | methyl | 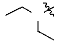 | |
| 83 | methyl | difluoromethyl | hydrogen | methyl | methyl | 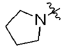 | Light brown oil |
| 84 | methyl | difluoromethyl | hydrogen | methyl | methyl | 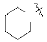 | |
| 85 | methyl | difluoromethyl | fluorine | methyl | methyl | 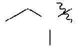 | |
| 86 | methyl | difluoromethyl | chlorine | methyl | methyl | 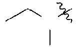 | |
| 87 | methyl | difluoromethyl | bromine | methyl | methyl | 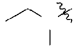 | |
| 88 | methyl | difluoromethyl | iodine | methyl | methyl | 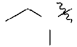 | |
| 89 | methyl | difluoromethyl | cyano | methyl | methyl | 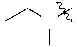 | |
| 90 | methyl | difluoromethyl | methyl | methyl | methyl | 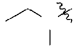 | |
| 91 | methyl | difluoromethyl | hydrogen | chlorine | methyl | 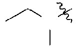 | |
| 92 | methyl | difluoromethyl | hydrogen | chlorine | methyl | 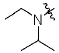 | |
| 93 | methyl | difluoromethyl | hydrogen | chlorine | methyl | 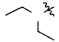 | |
| 94 | methyl | difluoromethyl | hydrogen | chlorine | methyl | 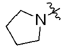 | |
| 95 | methyl | difluoromethyl | hydrogen | chlorine | methyl | 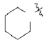 | |
| 96 | methyl | difluoromethyl | hydrogen | methyl | chlorine | 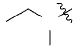 | |
| 97 | methyl | difluoromethyl | hydrogen | methyl | chlorine | 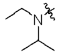 | |
| 98 | methyl | difluoromethyl | hydrogen | methyl | chlorine | 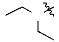 | |
| 99 | methyl | difluoromethyl | hydrogen | methyl | chlorine | 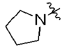 | |
| 100 | methyl | difluoromethyl | hydrogen | methyl | chlorine | 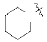 | |
| 101 | methyl | methyl | hydrogen | methyl | methyl | 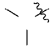 | Pale yellow solid |
| 102 | methyl | methyl | hydrogen | methyl | methyl | 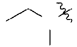 | Pale yellow solid |
| 103 | methyl | methyl | hydrogen | methyl | methyl | 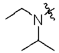 | |
| 104 | methyl | methyl | hydrogen | methyl | methyl | 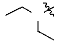 | Pale yellow solid |
| 105 | methyl | methyl | hydrogen | methyl | methyl | 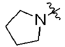 | |
| 106 | methyl | methyl | hydrogen | methyl | methyl | 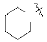 | |
| 107 | methyl | methyl | fluorine | methyl | methyl | 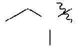 | |
| 108 | methyl | methyl | chlorine | methyl | methyl | 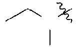 | |
| 109 | methyl | methyl | bromine | methyl | methyl | 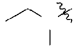 | |
| 110 | methyl | methyl | iodine | methyl | methyl | 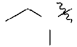 | |
| 111 | methyl | methyl | cyano | methyl | methyl | 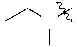 | |
| 112 | methyl | methyl | hydrogen | chlorine | methyl | 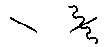 | Yellow solid (153.7-157.9 ) |
| 113 | methyl | methyl | hydrogen | chlorine | methyl | 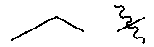 | Yellow solid (99.4-103.2) |
| 114 | methyl | methyl | hydrogen | chlorine | methyl | 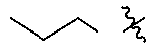 | |
| 115 | methyl | methyl | hydrogen | chlorine | methyl | 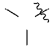 | Yellow solid |
| | | | | | | | (90.1-95.3) |
| 116 | methyl | methyl | hydrogen | chlorine | methyl | 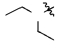 | Yellow solid (88.4-92.2) |
| 117 | methyl | methyl | hydrogen | chlorine | methyl | 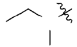 | Yellow solid (100.1-103.2 ) |
| 118 | methyl | methyl | hydrogen | chlorine | methyl | 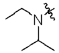 | |
| 119 | methyl | methyl | hydrogen | chlorine | methyl | 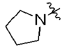 | Yellow solid (113.4-117.1) |
| 120 | methyl | methyl | hydrogen | chlorine | methyl | 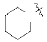 | |
| 121 | methyl | methyl | fluorine | chlorine | methyl | 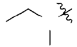 | |
| 122 | methyl | methyl | chlorine | chlorine | methyl | 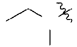 | |
| 123 | methyl | methyl | bromine | chlorine | methyl | 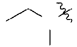 | |
| 124 | methyl | methyl | iodine | chlorine | methyl | 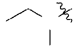 | |
| 125 | methyl | methyl | cyano | chlorine | methyl | 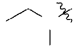 | |
| 126 | ethyl | methyl | hydrogen | chlorine | methyl | 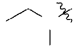 | |
| 127 | methyl | methyl | hydrogen | methyl | chlorine | 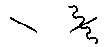 | Yellow solid (148.8-150.3 ) |
| 128 | methyl | methyl | hydrogen | methyl | chlorine | 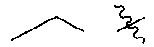 | Yellow solid (107.9-109.2 ) |
| 129 | methyl | methyl | hydrogen | methyl | chlorine | 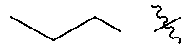 | Yellow solid (90.2-94.0) |
| 130 | methyl | methyl | hydrogen | methyl | chlorine | 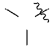 | Yellow solid (109.3-110.7 ) |
| 131 | methyl | methyl | hydrogen | methyl | chlorine | 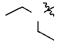 | Yellow solid (89.1-92.3) |
| 132 | methyl | methyl | hydrogen | methyl | chlorine | 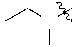 | Yellow solid (106.2-108.0 ) |
| 133 | methyl | methyl | hydrogen | methyl | chlorine | 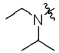 | |
| 134 | methyl | methyl | hydrogen | methyl | chlorine | 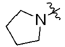 | Yellow solid (116.1-118.3) |
| 135 | methyl | methyl | hydrogen | methyl | chlorine | 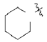 | |
| 136 | methyl | methyl | fluorine | methyl | chlorine | 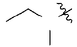 | |
| 137 | methyl | methyl | chlorine | methyl | chlorine | 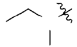 | |
| 138 | methyl | methyl | bromine | methyl | chlorine | 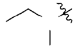 | |
| 139 | methyl | methyl | iodine | methyl | chlorine | 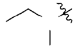 | |
| 140 | methyl | methyl | cyano | methyl | chlorine | 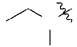 | |
| 141 | methyl | methyl | methyl | methyl | methyl | 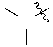 | |
| 142 | methyl | methyl | methyl | methyl | methyl | 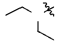 | |
| 143 | methyl | methyl | methyl | methyl | methyl | 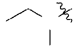 | Yellow oily liquid |
| 144 | methyl | methyl | methyl | methyl | methyl | 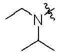 | |
| 145 | methyl | methyl | methyl | methyl | methyl | 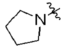 | Yellow oily liquid |
| 146 | methyl | methyl | methyl | methyl | methyl | 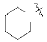 | |
| 147 | methyl | cyclopropyl | hydrogen | methyl | methyl | 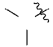 | |
| 148 | methyl | cyclopropyl | hydrogen | methyl | methyl | 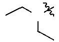 | |
| 149 | methyl | cyclopropyl | hydrogen | methyl | methyl | 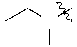 | Red oily liquid |
| 150 | methyl | cyclopropyl | hydrogen | methyl | methyl | 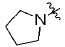 | Yellow oily liquid |
| 151 | methyl | cyclopropyl | hydrogen | methyl | methyl | 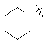 | |
| 152 | methyl | cyano | hydrogen | methyl | methyl | 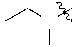 | |
| 153 | methyl | cyano | hydrogen | methyl | methyl | 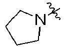 | |
| 154 | methyl | cyano | hydrogen | methyl | methyl | 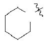 | |
| 155 | methyl | phenyl | hydrogen | methyl | methyl | 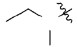 | Yellow oily liquid |
| 156 | methyl | phenyl | hydrogen | methyl | methyl | 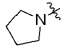 | Yellow solid (44.2-47.8) |
| 157 | methyl | phenyl | hydrogen | methyl | methyl | 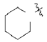 | Yellow oily liquid |
| 158 | methyl | phenyl | hydrogen | methyl | methyl | 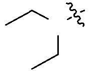 | Yellow oily liquid |
| 159 | methyl | 4-chlorophenyl | hydrogen | methyl | methyl | 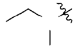 | |
| 160 | methyl | 4-chlorophenyl | hydrogen | methyl | methyl | 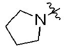 | |
| 161 | methyl | 4-chlorophenyl | hydrogen | methyl | methyl | 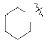 | |
| 162 | methyl | trifluoromethyl | hydrogen | methyl | hydrogen | 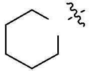 | Yellow oily liquid |
| 163 | methyl | trifluoromethyl | hydrogen | methyl | hydrogen | 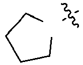 | Yellow oily liquid |
| 164 | methyl | trifluoromethyl | hydrogen | methyl | hydrogen | 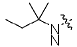 | Yellow oily liquid |
| 165 | methyl | trifluoromethyl | hydrogen | methyl | hydrogen | 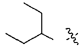 | Yellow oily liquid |
| 166 | methyl | trifluoromethyl | hydrogen | methyl | hydrogen | 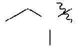 | Yellow oily liquid |
| 167 | methyl | trifluoromethyl | hydrogen | methyl | hydrogen | 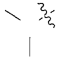 | Yellow oily liquid |
| 168 | methyl | trifluoromethyl | hydrogen | methyl | hydrogen | 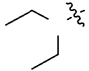 | Yellow oily liquid |
| 169 | methyl | trifluoromethyl | hydrogen | methoxy | methyl | 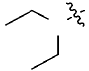 | Yellow oily liquid |
| 170 | methyl | trifluoromethyl | hydrogen | methoxy | methyl | 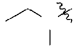 | Yellow oily liquid |
| 171 | methyl | trifluoromethyl | hydrogen | methoxy | methyl | 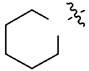 | Red solid (101.3-102.4 ) |
| 172 | phenyl | trifluoromethyl | hydrogen | methyl | hydrogen | 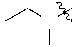 | Yellow oily liquid |
| 173 | phenyl | trifluoromethyl | hydrogen | methyl | hydrogen | 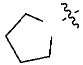 | Yellow oily liquid |
| 174 | phenyl | trifluoromethyl | hydrogen | methyl | hydrogen | 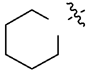 | Yellow oily liquid |
| 175 | ethyl | trifluoromethyl | hydrogen | methyl | methyl | 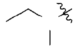 | Yellow oily liquid |
| 176 | ethyl | trifluoromethyl | hydrogen | methyl | methyl | 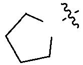 | Yellow oily liquid |
| 177 | ethyl | trifluoromethyl | hydrogen | methyl | methyl | 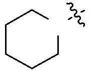 | Yellow oily liquid |
| 178 | ethyl | trifluoromethyl | hydrogen | methyl | methyl | 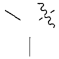 | Yellow oily liquid |
| 179 | ethyl | trifluoromethyl | hydrogen | methyl | methyl | 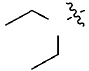 | Yellow oily liquid |
| 180 | ethyl | trifluoromethyl | hydrogen | methyl | methyl | 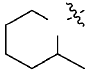 | Red oily liquid |
| 181 | ethyl | trifluoromethyl | hydrogen | methyl | methyl | 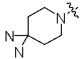 | Red solid (74.5-75.2) |
| 182 | ethyl | trifluoromethyl | hydrogen | methyl | methyl | 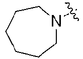 | Red oily liquid |
| 183 | ethyl | trifluoromethyl | hydrogen | methyl | methyl | 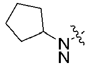 | Yellow oily liquid |
| 184 | ethyl | trifluoromethyl | hydrogen | chlorine | methyl | 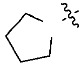 | Yellow oily liquid |
| 185 | ethyl | trifluoromethyl | hydrogen | chlorine | methyl | 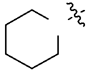 | Yellow oily liquid |
| 186 | ethyl | trifluoromethyl | hydrogen | chlorine | methyl | 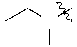 | Yellow oily liquid |
| 187 | ethyl | trifluoromethyl | hydrogen | chlorine | methyl | 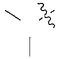 | Yellow solid (66.8-67.5) |
| 188 | ethyl | trifluoromethyl | hydrogen | chlorine | methyl | 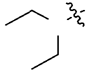 | Yellow solid (75.3-76.4) |
| 189 | ethyl | trifluoromethyl | hydrogen | chlorine | methyl | 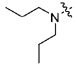 | Yellow oily liquid |
| 190 | ethyl | trifluoromethyl | hydrogen | chlorine | methyl | 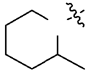 | Red oily liquid |
| 191 | ethyl | trifluoromethyl | hydrogen | chlorine | methyl | 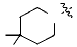 | Red oily liquid |
| 192 | ethyl | trifluoromethyl | hydrogen | chlorine | methyl | 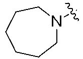 | Yellow oily liquid |
| 193 | ethyl | trifluoromethyl | hydrogen | methyl | chlorine | 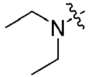 | Yellow oily liquid |
| 194 | ethyl | trifluoromethyl | hydrogen | methyl | chlorine | 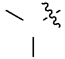 | Yellow oily liquid |
| 195 | ethyl | trifluoromethyl | hydrogen | methyl | chlorine | 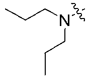 | Yellow oily liquid |
| 196 | ethyl | trifluoromethyl | hydrogen | methyl | chlorine | 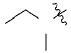 | Yellow oily liquid |
| 197 | ethyl | trifluoromethyl | hydrogen | methyl | chlorine | 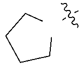 | White solid (74.1-75.6) |
| 198 | ethyl | trifluoromethyl | hydrogen | methyl | chlorine | 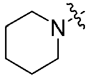 | White solid (75.3-76.5) |
| 199 | *N*-propyl | trifluoromethyl | hydrogen | methyl | methyl | 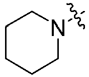 | Yellow solid (75.5-76.3) |
| 200 | *N*-propyl | trifluoromethyl | hydrogen | methyl | methyl | 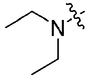 | Yellow solid (78.1-78.9) |
| 201 | *N*-propyl | trifluoromethyl | hydrogen | methyl | methyl | 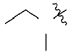 | Yellow solid (76.6-77.2) |
| 202 | *N*-propyl | trifluoromethyl | hydrogen | chlorine | methyl | 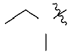 | Yellow oily liquid |
| 203 | *N*-propyl | trifluoromethyl | hydrogen | chlorine | methyl | 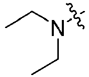 | Yellow solid (76.3-77.2) |
| 204 | *N*-propyl | trifluoromethyl | hydrogen | chlorine | methyl | 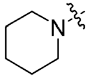 | Yellow oily liquid |
| 205 | *N*-propyl | trifluoromethyl | hydrogen | chlorine | methyl | 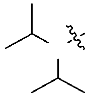 | Yellow oily liquid |
| 206 | *N*-propyl | trifluoromethyl | hydrogen | chlorine | methyl | 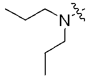 | Yellow oily liquid |
| 207 | isopropyl | trifluoromethyl | hydrogen | methyl | methyl | 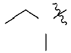 | Yellow solid (63.2-64.5) |
| 208 | isopropyl | trifluoromethyl | hydrogen | methyl | methyl | 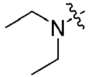 | Yellow solid (70.8-71.5) |
| 209 | isopropyl | trifluoromethyl | hydrogen | methyl | methyl | 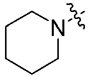 | Yellow oily liquid |
| 210 | isopropyl | trifluoromethyl | hydrogen | methyl | methyl | 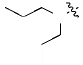 | Yellow solid (78.9-79.7) |
| 211 | isopropyl | trifluoromethyl | hydrogen | methyl | methyl | 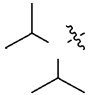 | Yellow oily liquid |
| 212 | isopropyl | trifluoromethyl | hydrogen | chlorine | methyl | 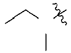 | Yellow oily liquid |
| 213 | isopropyl | trifluoromethyl | hydrogen | chlorine | methyl | 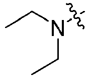 | Yellow solid (75.3-76.6) |
| 214 | isopropyl | trifluoromethyl | hydrogen | chlorine | methyl | 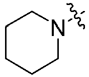 | Yellow solid (75.3-76.1) |
| 215 | *Tert-*butyl | trifluoromethyl | hydrogen | methyl | methyl | 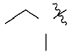 | Yellow oily liquid |
| 216 | *Tert*-butyl | trifluoromethyl | hydrogen | methyl | methyl | 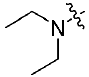 | Yellow solid (66.7-67.9) |
| 217 | *Tert*-butyl | trifluoromethyl | hydrogen | methyl | methyl | 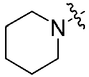 | Yellow solid (89.3-90.5) |
| 218 | *Tert-*butyl | trifluoromethyl | hydrogen | methyl | methyl | 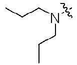 | Yellow oily liquid |
| 219 | *Tert-*butyl | trifluoromethyl | hydrogen | methyl | methyl | 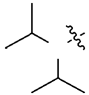 | Yellow oily liquid |
| 220 | *Tert-*butyl | trifluoromethyl | hydrogen | chlorine | methyl | 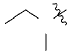 | Yellow oily liquid |
| 221 | *Tert*-butyl | trifluoromethyl | hydrogen | chlorine | methyl | 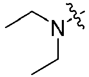 | Yellow solid (76.7-77.4) |
| 222 | *Tert-*butyl | trifluoromethyl | hydrogen | chlorine | methyl | 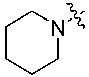 | Yellow solid (93.1-94.5) |
| 223 | *Tert-*butyl | trifluoromethyl | hydrogen | chlorine | methyl | 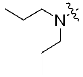 | Purple oily liquid |
| 224 | *Tert-*butyl | trifluoromethyl | hydrogen | chlorine | methyl | 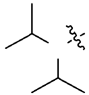 | Yellow oily liquid |
| 225 | trifluoroeth yl | trifluoromethyl | hydrogen | chlorine | methyl | 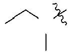 | Yellow oily liquid |
| 226 | trifluoroeth yl | trifluoromethyl | hydrogen | chlorine | methyl | 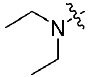 | Yellow oily liquid |
| 227 | trifluoroeth yl | trifluoromethyl | hydrogen | chlorine | methyl | 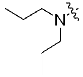 | Yellow oily liquid |
| 228 | trifluoroeth yl | trifluoromethyl | hydrogen | chlorine | methyl | 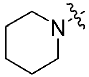 | Yellow oily liquid |
| 229 | trifluoroeth yl | trifluoromethyl | hydrogen | chlorine | methyl | 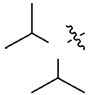 | Yellow oily liquid |
| 230 | cyclopropyl | trifluoromethyl | hydrogen | chlorine | methyl | 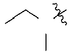 | Yellow oily liquid |
| 231 | cyclopropyl | trifluoromethyl | hydrogen | chlorine | methyl | 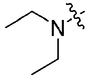 | Yellow oily liquid |
| 232 | cyclopropyl | trifluoromethyl | hydrogen | chlorine | methyl | 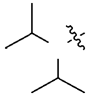 | Yellow oily liquid |
| 233 | cyclopropyl | trifluoromethyl | hydrogen | chlorine | methyl | 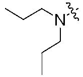 | Yellow oily liquid |
| 234 | cyclopropyl | trifluoromethyl | hydrogen | chlorine | methyl | 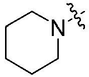 | Yellow oily liquid |
| 235 | methyl | trifluoromethyl | methyl | methyl | methyl | 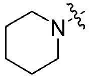 | Yellow oily liquid |
| 236 | methyl | trifluoromethyl | methyl | methyl | methyl | 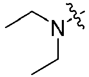 | Yellow oily liquid |
| 237 | methyl | trifluoromethyl | methyl | methyl | methyl | 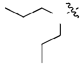 | Yellow oily liquid |
| 238 | methyl | trifluoromethyl | methyl | chlorine | methyl | 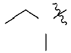 | Yellow oily liquid |
| 239 | methyl | trifluoromethyl | methyl | chlorine | methyl | 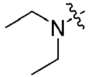 | Yellow oily liquid |
| 240 | methyl | trifluoromethyl | methyl | chlorine | methyl | 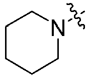 | Yellow oily liquid |
| 241 | methyl | trifluoromethyl | methyl | chlorine | methyl | 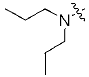 | Yellow oily liquid |
| 242 | ethyl | methyl | hydrogen | methyl | methyl | 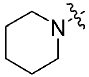 | Yellow oily liquid |
| 243 | ethyl | methyl | hydrogen | methyl | methyl | 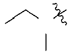 | Yellow oily liquid |
| 244 | ethyl | methyl | hydrogen | methyl | methyl | 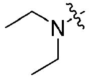 | Yellow oily liquid |
| 245 | ethyl | methyl | hydrogen | methyl | methyl | 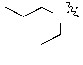 | Yellow oily liquid |
| 246 | ethyl | methyl | hydrogen | chlorine | methyl | 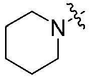 | Yellow oily liquid |
| 247 | ethyl | methyl | hydrogen | chlorine | methyl | 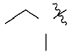 | Yellow oily liquid |
| 248 | ethyl | methyl | hydrogen | chlorine | methyl | 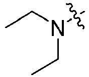 | Yellow oily liquid |
| 249 | ethyl | methyl | hydrogen | chlorine | methyl | 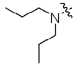 | Yellow oily liquid |
| 250 | isopropyl | trifluoromethyl | hydrogen | methyl | chlorine | 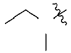 | White solid (77.5-78.7) |
| 251 | isopropyl | trifluoromethyl | hydrogen | methyl | chlorine | 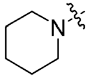 | White solid (76.3-86.9) |
| 252 | isopropyl | trifluoromethyl | hydrogen | methyl | chlorine | 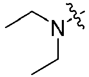 | White solid (75.3-76.4) |
| 253 | isopropyl | trifluoromethyl | hydrogen | methyl | chlorine | 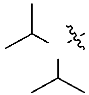 | White solid (67.8-69.3) |
| 254 | isobutyl | trifluoromethyl | hydrogen | methyl | methyl | 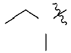 | Yellow oily liquid |
| 255 | isobutyl | trifluoromethyl | hydrogen | methyl | methyl | 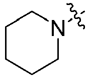 | Yellow oily liquid |
| 256 | isobutyl | trifluoromethyl | hydrogen | methyl | methyl | 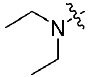 | |
| 257 | isobutyl | trifluoromethyl | hydrogen | methyl | methyl | 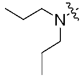 | |
| 258 | isobutyl | trifluoromethyl | hydrogen | methyl | methyl | 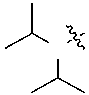 | |
| 259 | isobutyl | trifluoromethyl | hydrogen | chlorine | methyl | 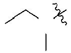 | Yellow oily liquid |
| 260 | isobutyl | trifluoromethyl | hydrogen | chlorine | methyl | 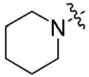 | Yellow oily liquid |
| 261 | isobutyl | trifluoromethyl | hydrogen | chlorine | methyl | 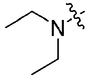 | Yellow oily liquid |
| 262 | isobutyl | trifluoromethyl | hydrogen | chlorine | methyl | 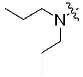 | Yellow oily liquid |
| 263 | isobutyl | trifluoromethyl | hydrogen | chlorine | methyl | 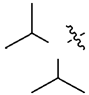 | Yellow oily liquid |

Further preferably, in Formula I,
R₁ is selected from methyl, ethyl, n-propyl, isopropyl, *tert-*butyl*,* isobutyl or cyclopropyl group, which is substituted with at least one R₈;
R₂ is selected from methyl, difluoromethyl or trifluoromethyl group;
R₃ is selected from hydrogen, fluorine, chlorine, cyano or methyl group;
R₄ is selected from methyl, methoxy or chlorine group;
R₅ is selected from methyl or chlorine group;
R₆ is selected from methyl, ethyl, *n*-propyl or isopropyl group;
R₇ is selected from hydrogen, methyl, ethyl, *n*-propyl or isopropyl group;
or R₆ and R₇, together with the nitrogen atom to which they are attached, form tetrahydropyrrole, piperidine or azacyclooctane, and the tetrahydropyrrole, piperidine or azacyclooctane may be substituted with identical or different R₈;
R₈ is selected from hydrogen, methyl or fluorine group.

Further preferably, in Formula I,
R₁ is selected from methyl, ethyl, *n*-propyl, isopropyl or *tert-butyl* group;
R₂ is selected from trifluoromethyl group;
R₃ is selected from hydrogen or methyl group;
R₄ is selected from methyl or chlorine group;
R₅ is selected from methyl or chlorine group;
R₆ is selected from ethyl group;
R₇ is selected from methyl group;
or R₆ and R₇, together with the nitrogen atom to which they are attached, form piperidine, and the piperidine may be substituted with identical or different R₈;
R₈ is selected from hydrogen or methyl group.

Most preferably, the pyrazol-5-ether compound is any one of the following compounds:

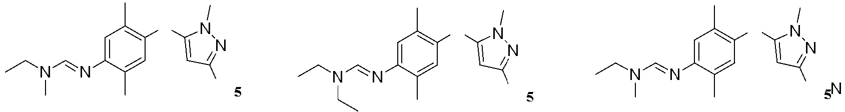

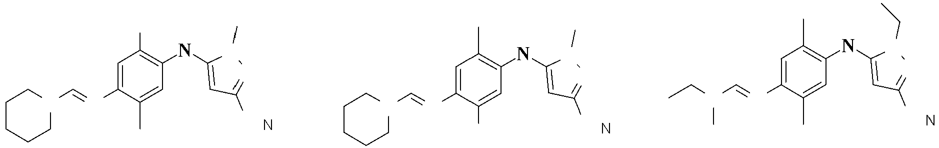

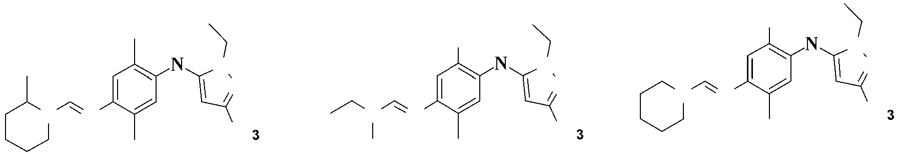

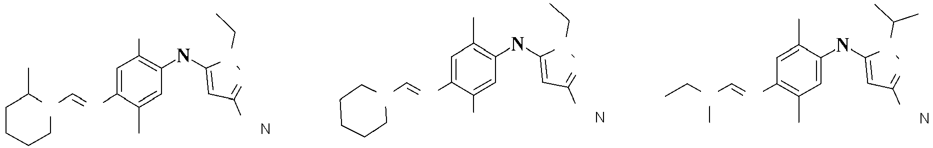

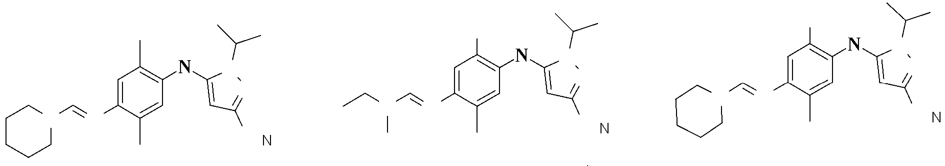

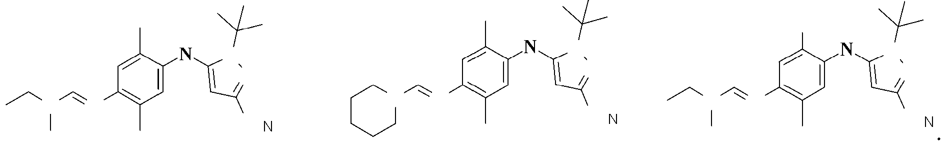

Alkyl in the present application represents a straight-chain or branched alkyl group, for example, methyl, ethyl, *n*-propyl, isopropyl, *n*-butyl, isobutyl, *sec*-butyl*, tert-*butyl, *n*-pentyl, isopentyl, *n*-hexyl, and the like. Haloalkyl represents an alkyl group substituted by one or more halogen atoms. Alkoxyl represents an alkyl group which is attached to an oxygen atom at its terminal position, for example, methoxy, ethoxy, n-propoxy, isopropoxy, *tert*-butxoyl, and the like. Haloalkoxy represents alkoxy substituted by one or more halogen atoms. Halogen refers to F, Cl, Br or I.

As used herein, the term "C1-C6 alkyl" represents a straight-chain or branched alkyl group having 1 to 6 carbon atoms, including but not limited to, methyl, ethyl, *n*-propyl, isopropyl, *n*-butyl, isobutyl, *sec-*butyl*, tert-*butyl*, n*-pentyl, isopentyl, *n*-hexyl, and the like. The term "C1-C6 alkoxy" represents a straight-chain or branched alkoxy group having 1 to 6 carbon atoms, including, but not limited to, methoxy, ethoxy, n-propoxy, isopropoxy, *tert*-butxoy, and the like. The term "C1-C12 alkoxy" has a similar meaning.

As used herein, the term "C3-C8 cycloalkyl" represents a cyclic alkyl group having 3 to 8 carbon atoms in the ring, including, but not limited to, cyclopropyl, cyclobutyl, cyclopentyl, cyclohexyl, cycloheptyl, cyclooctyl, and the like.

In the present application, C2-C12, C1-C6, C3-C8, and the like in front of a specific group refer to the number of carbon atoms contained in the group. For example, C2-C12 represents a group containing 2, 3, 4, 5, 6, 7, 8, 9, 10, 11 or 12 carbon atoms, C1-C6 represents a group containing 1, 2, 3, 4, 5 or 6 carbon atoms, C3-C8 represents a group containing 3, 4, 5, 6, 7 or 8 carbon atoms, C2-C4 represents a group containing 2, 3 or 4 carbon atoms, and the like.

In the present application, " 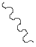 " in the group represents a position where the group is attached.

In a second aspect, the present application provides a method for preparing the pyrazol-5-ether compound described in the first aspect, and the preparation method includes the following step:
reacting a compound represented by Formula II with a compound represented by Formula III to obtain the pyrazol-5-ether compound represented by Formula I, and the reaction formula is as follows: 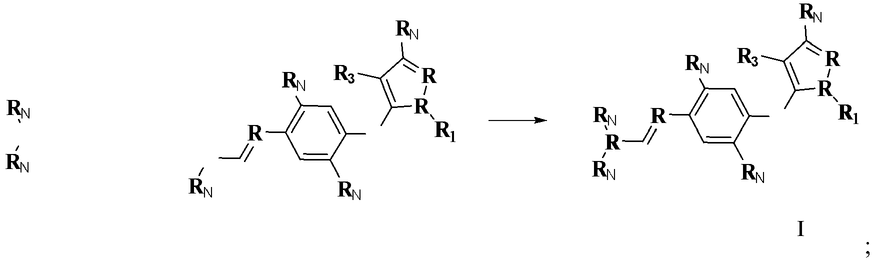
wherein, the definitions of R₁, R₂, R₃, R₄, R₅, R₆, and R₇ are as described above, which will not be repeated herein; and R₉ is selected from C1-C6 alkyl.

Preferably, the molar ratio of the compound represented by Formula II to the compound represented by Formula III is 0.5:1-2:1, for example, 0.5:1, 0.8:1, 1:1, 1.2:1, 1.5:1, 1.8:1 or 2:1.

Preferably, the reaction is carried out in a solvent which is any one or a combination of at least two of dichloromethane, chloroform, ethyl acetate, toluene, acetonitrile, tetrahydrofuran, dioxane, ethanol, methanol, N,N-dimethylformamide or dimethylsulfoxide.

Preferably, the reaction is carried out at a temperature which is greater than or equal to room temperature and less than or equal to the boiling point of the reaction solvent, for example, 20 °C, 25 °C, 30 °C, 35 °C, 40 °C, 45 °C, 50 °C, 60 °C, 70 °C, 75 °C, 80 °C, 85 °C or 90 °C, or the reaction is carried out at the boiling point of the solvent, i.e., the reaction is carried out in a reflux state.

Preferably, the reaction is carried out for 0.5 hours to 48 hours, for example, 0.5 hours, 1 hour, 3 hours, 5 hours, 8 hours, 10 hours, 12 hours, 15 hours, 18 hours, 20 hours, 23 hours, 25 hours, 28 hours, 30 hours, 33 hours, 35 hours, 38 hours, 40 hours, 44 hours or 48 hours.

Preferably, the compound represented by Formula III is prepared in the following method: reacting a compound represented by Formula IV with a compound represented by Formula V in the presence of a catalyst to obtain the compound represented by Formula III, and the reaction formula is as follows: 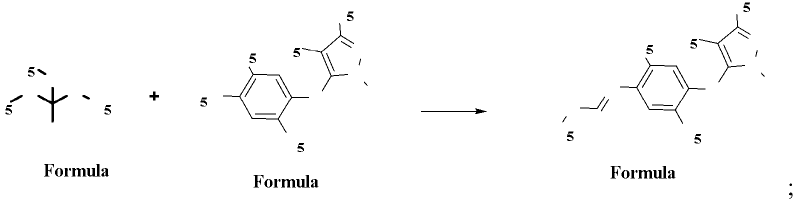 wherein, the definitions of R₁, R₂, R₃, R₄, R₅, and R₉ are as described above, which will not be repeated herein.

Preferably, in the preparation of the compound represented by Formula III, the reaction is carried out in the presence of an acidic substance, and the acidic substance is an organic acid and/or an inorganic acid.

The organic acid is any one or a combination of at least two of *p*-toluenesulfonic acid, methanesulfonic acid, trifluoroacetic acid, and acetic acid.

The inorganic acid is any one or a combination of at least two of hydrochloric acid, hydrobromic acid, sulfuric acid, zinc chloride, ammonium chloride, and ferric trichloride.

Preferably, no solvent may be added in the preparation of the compound represented by Formula III.

Preferably, in the preparation of the compound represented by Formula III, the reaction is carried out in a suitable solvent, and the suitable solvent is any one or a combination of at least two of dichloromethane, chloroform, ethyl acetate, toluene, acetonitrile, tetrahydrofuran, 1,4-dioxane, methanol, ethanol, isopropanol, n-butanol, water, N,N-dimethylformamide or dimethylsulfoxide.

Preferably, in the preparation of the compound represented by Formula III, the reaction is carried out at a temperature which is greater than or equal to 0 °C and less than or equal to the boiling point of the reaction solvent.

Preferably, in the preparation of the compound represented by Formula III, the reaction is carried out for 0.5 hours to 48 hours.

Preferably, the compound represented by Formula IV is prepared in the following: reducing a compound represented by Formula VI in the presence of a reductant to obtain the compound represented by Formula IV, and the reaction formula is as follows: 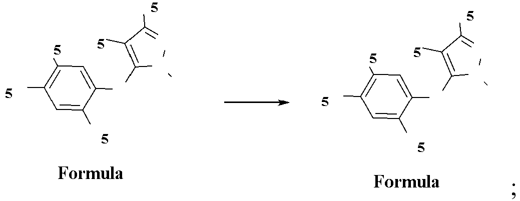 wherein, the definitions of R₁, R₂, R₃, R₄, and R₅ are as described above, which will not be repeated herein.

Preferably, in the preparation of the compound represented by Formula IV, the reductant is any one or a combination of at least two of hydrogen, hydrazine hydrate, iron powder, zinc powder, stannous chloride or sodium dithionite.

Preferably, in the preparation of the compound represented by Formula IV, the reaction is carried out in the presence of a catalyst, and the catalyst is preferably any one or a combination of at least two of palladium on carbon, palladium dioxide, Raney nickel, ferric trichloride or basic iron oxide.

Preferably, in the preparation of the compound represented by Formula IV, the reaction is carried out in a solvent which is any one or a combination of at least two of dichloromethane, chloroform, ethyl acetate, toluene, acetonitrile, tetrahydrofuran, dioxane, ethanol, methanol, *N,N*-dimethylformamide, a saturated ammonium chloride aqueous solution, acetic acid, hydrochloric acid, water or dimethylsulfoxide.

Preferably, in the preparation of the compound represented by Formula IV, the reaction is carried out at a temperature which is greater than or equal to 0 °C and less than or equal to the boiling point of the reaction solvent.

Preferably, in the preparation of the compound represented by Formula IV, the reaction is carried out for 0.5 hours to 48 hours.

Preferably, the compound represented by Formula VI is prepared in the following method: reacting a compound represented by Formula VII with a compound represented by Formula VIII to obtain the compound represented by Formula VI, and the reaction formula is as follows: 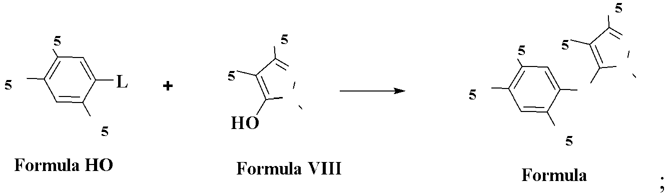 wherein, L is fluorine or chlorine; in addition, the definitions of R₁, R₂, R₃, R₄, and R₅ are as described above, which will not be repeated herein.

Preferably, the molar ratio of the compound represented by Formula VII to the compound represented by Formula VIII is 1:1-3:1.

Preferably, in the preparation of the compound represented by Formula VI, the reaction is carried out in the presence of a basic substance, and the basic substance is an organic base and/or an inorganic base.

Preferably, the organic base is any one or a combination of at least two of triethylamine, *N,N*-dimethylaniline, pyridine, sodium methoxide, sodium ethoxide, sodium tert-butoxide or potassium tert-butoxide.

Preferably, the inorganic base is any one or a combination of at least two of sodium hydroxide, potassium hydroxide, sodium carbonate, potassium carbonate or sodium hydride.

Preferably, in the preparation of the compound represented by Formula VI, the reaction is carried out in a solvent which is any one or a combination of at least two of dichloromethane, chloroform, acetone, toluene, acetonitrile, tetrahydrofuran, dioxane, methanol, ethanol, N,N-dimethylformamide, dimethylsulfoxide or hexamethylphosphoramide.

Preferably, in the preparation of the compound represented by Formula VI, the reaction is carried out at a temperature which is greater than or equal to 0 °C and less than or equal to the boiling point of the reaction solvent, for example, 0 °C, 20 °C, 25 °C, 30 °C, 35 °C, 40 °C, 45 °C, 50 °C, 60 °C, 70 °C, 75 °C, 80 °C, 85 °C or 90 °C, or the reaction is carried out at the boiling point of the solvent, i.e., the reaction is carried out in a reflux state.

Preferably, in the preparation of the compound represented by Formula VI, the reaction is carried out for 0.5 hours to 48 hours.

In the present application, some of the compounds represented by Formula VIII used as raw materials are commercially available reagents, and some compounds can be synthesized with reference to literature, such as Chinese Journal of Organic Chemistry (2015), 35 (1), 100-108*;* Pest Management Science (2014), 70 (8), 1207-1214; Molecules (2022), 27 (19), 6274; and New Journal of Chemistry (2019), 43 (7), 3000-3010.

In the present application, Table 2 lists representative compounds of the intermediate compounds represented by Formula VI of the present application, but the present application is not limited thereto:

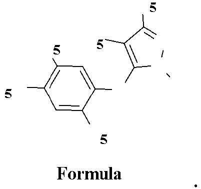

**Table 2**

| No. | Structural formula | Character | No. | Structural formula | Character |
|---|---|---|---|---|---|
| VI1 | 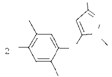 | Yellow solid | VI27 | 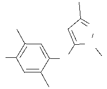 | Orange-yellow solid |
| VI2 | 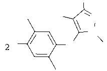 | | VI28 | 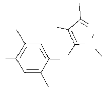 | |
| VI3 | 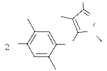 | | VI29 | 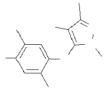 | |
| VI4 | 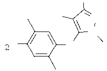 | | VI30 | 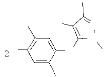 | |
| VI5 | 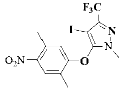 | | VI31 | 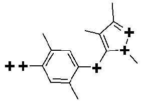 | |
| VI6 | 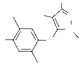 | | VI32 | 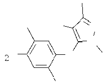 | |
| VI7 | 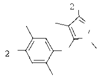 | | VI33 | 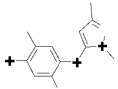 | Yellow solid |
| VI8 | 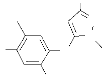 | | VI34 | 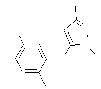 | Yellow solid |
| VI9 | 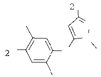 | Light brown oil | VI35 | 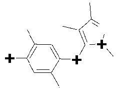 | Yellow solid |
| VI10 | 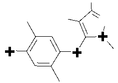 | | VI36 | 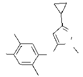 | Yellow solid |
| VI11 | 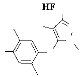 | | VI37 | 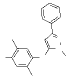 | Yellow solid |
| VI12 | 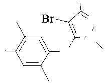 | | VI38 | 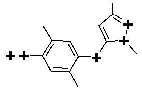 | |
| VI13 | 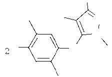 | | VI39 | 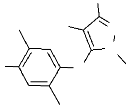 | |
| VI14 | 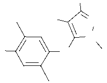 | | VI40 | 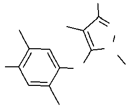 | |
| VI15 | 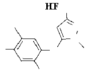 | | VI41 | 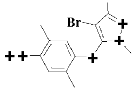 | |
| VI16 | 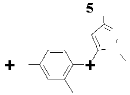 | Yellow solid | VI42 | 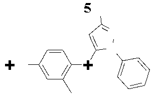 | Yellow solid |
| VI17 | 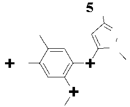 | Yellow solid | VI43 | 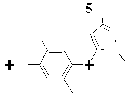 | Yellow solid |
| VI18 | 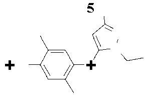 | Yellow solid | VI44 | 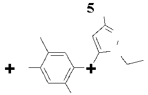 | Yellow solid |
| VI19 | 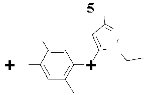 | Yellow solid | VI45 | 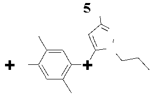 | Yellow solid |
| VI20 | 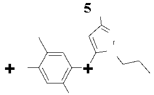 | Yellow solid | VI46 | 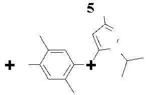 | Yellow solid |
| VI21 | 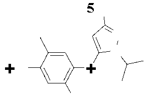 | Yellow solid | VI47 | 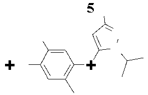 | Yellow solid |
| VI22 | 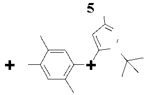 | Yellow solid | VI48 | 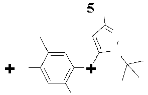 | Yellow solid |
| VI23 | 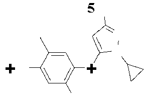 | Yellow solid | VI49 | 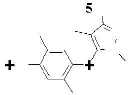 | Yellow solid |
| VI24 | 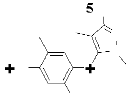 | Yellow solid | VI50 | 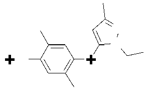 | Yellow solid |
| VI25 | 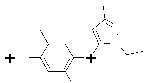 | Yellow solid | VI51 | 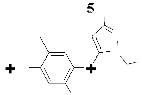 | Yellow solid |
| VI26 | 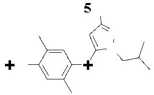 | Yellow solid | VI52 | 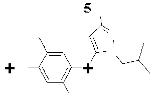 | Yellow solid |

In the present application, Table 3 lists representative compounds of the intermediate compounds represented by Formula IV of the present application, but the present application is not limited thereto:

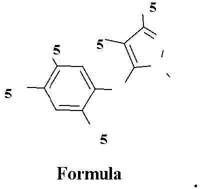

**Table 3**

| No. | Structural formula | Character | No. | Structural formula | Character |
|---|---|---|---|---|---|
| IV1 | 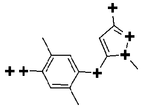 | Yellow solid | IV27 | 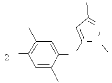 | Pale yellow solid |
| IV2 | 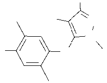 | | IV28 | 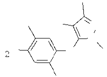 | |
| IV3 | 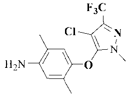 | | IV29 | 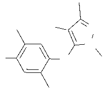 | |
| IV4 | 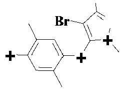 | | IV30 | 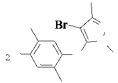 | |
| IV5 | 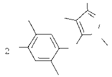 | | IV31 | 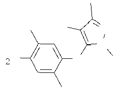 | |
| IV6 | 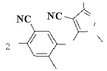 | | IV32 | 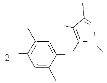 | |
| IV7 | 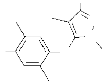 | | IV33 | 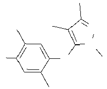 | Yellow solid |
| IV8 | 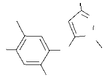 | | IV34 | 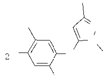 | Yellow solid |
| IV9 | 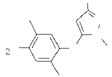 | Light brown oil | IV354 | 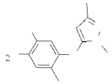 | Yellow solid |
| IV10 | 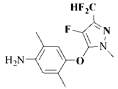 | | IV36 | 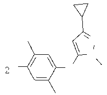 | Yellow solid |
| IV11 | 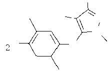 | | IV37 | 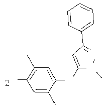 | Red solid |
| IV12 | 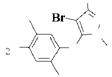 | | IV38 | 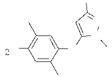 | |
| IV13 | 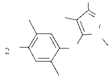 | | IV39 | 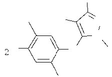 | |
| IV14 | 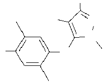 | | IV40 | 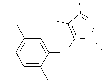 | |
| IV15 | 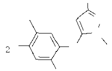 | | IV41 | 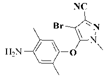 | |
| IV16 | 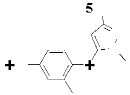 | Brown oily substance | IV42 | 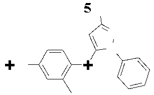 | Brown oily substance |
| IV17 | 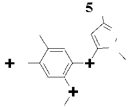 | Brown oily substance | IV43 | 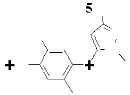 | Brown oily substance |
| IV18 | 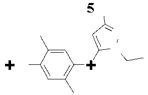 | Brown oily substance | IV44 | 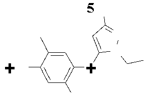 | Brown oily substance |
| IV19 | 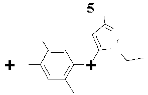 | Brown oily substance | IV45 | 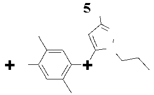 | Brown oily substance |
| IV20 | 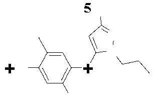 | Brown oily substance | IV46 | 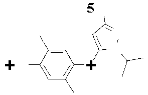 | Brown oily substance |
| IV21 | 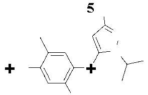 | Brown oily substance | IV47 | 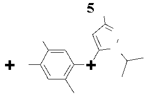 | Brown oily substance |
| IV22 | 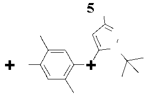 | Brown oily substance | IV48 | 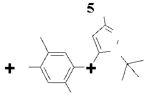 | Brown oily substance |
| IV23 | 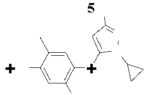 | Brown oily substance | IV49 | 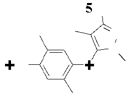 | Brown oily substance |
| IV24 | 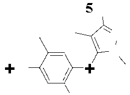 | Brown oily substance | IV50 | 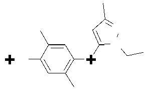 | Brown oily substance |
| IV25 | 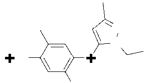 | Brown oily substance | IV51 | 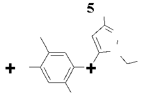 | Brown oily substance |
| IV26 | 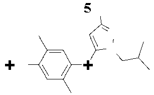 | Brown oily substance | IV52 | 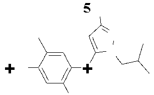 | Brown oily substance |

In a third aspect, the present application provides the use of the pyrazol-5-ether compound described in the first aspect in the prevention and treatment of plant diseases.

The pyrazol-5-ether compound of the present application has unexpectedly high fungicidal activity and has a good control effect on plant diseases.

In the present application, the plant diseases include diseases caused by *Oomycetes, Ascomycetes, Basidiomycetes* or *Deuteromycetes.*

Preferably, the plant diseases include, but is not limited to, wheat rust, wheat powdery mildew, wheat scab, wheat root rot, wheat sheath blight, wheat take-all, wheat glume blotch, cucumber downy mildew, cucumber powdery mildew, melon powdery mildew, bitter gourd powdery mildew, cucumber anthracnose, cucumber fusarium wilt, cucumber gray mold, grape downy mildew, tomato early blight, tomato late blight, rice sheath blight, rice blast, watermelon gummy stem blight, peanut scab, peanut black spot, citrus scab, pepper root rot, cotton verticillium wilt, cotton fusarium wilt, rape blackleg, rape sclerotinia stem rot, pear scab, ginseng rust rot, corn rust, Curvularia lunata, corn northern leaf blight, mango stem-end rot, apple ring rot, apple rot, banana leaf spot, soybean rust or potato late blight.

Preferably, the plant diseases include wheat rust, wheat powdery mildew, wheat scab, cucumber powdery mildew, cucumber downy mildew or soybean rust.

In a fourth aspect, the present application provides a fungicide composition. The fungicide composition includes an active constituent and an agrochemically acceptable carrier, and the active constituent is the pyrazol-5-ether compound described above.

The fungicide composition of the present application can be used in the fields of agriculture, forestry, health, and the like.

Preferably, the weight percentage of the active constituent in the fungicide composition is 1% to 99%, for example, 1%, 3%, 5%, 8%, 10%, 15%, 18%, 20%, 25%, 30%, 35%, 40%, 45%, 50%, 55%, 60%, 65%, 70%, 75%, 80%, 85%, 90%, 95% or 99%.

Preferably, the agrochemically acceptable carrier includes surfactants.

In the present application, the surfactants are ionic surfactants or non-ionic surfactants.

The surfactants include emulsifiers, dispersants or wetting agents. The emulsifiers may be polyoxyethylene fatty acid ester, polyoxyethylene fatty alcohol ether, polyoxyethylene fatty amine and commercially available emulsifiers (such as pesticide emulsifier 2201B, pesticide emulsifier 0203B, pesticide emulsifier 100#, pesticide emulsifier 500#, pesticide emulsifier 600#, pesticide emulsifier 600-2#, pesticide emulsifier 1601, pesticide emulsifier 2201, pesticide emulsifier NP-10, pesticide emulsifier NP-15, pesticide emulsifier 507#, pesticide emulsifier OX-635, pesticide emulsifier OX-622, pesticide emulsifier OX-653, pesticide emulsifier OX-667, and pesticide emulsifier 36#). The dispersants include sodium lignosulfonate, Nekal BX, calcium lignosulfonate, and methylnaphthalene sulfonate formaldehyde condensate. The wetting agents include sodium lauryl sulfate, sodium dodecyl benzene sulfonate, and sodium alkylnaphthalenesulfonate.

Preferably, the agrochemically acceptable carrier includes solid carriers and/or liquid carriers.

Preferably, the solid carriers include natural or synthetic clays and silicates, for example, natural silica and diatomaceous earth; magnesium silicates such as talc; magnesium aluminum silicates such as kaolinite, kaolin, montmorillonite, and mica; precipitated silica; calcium carbonate; light calcium carbonate; calcium sulfate; limestone; sodium sulfate; and amine salts such as ammonium sulfate and hexamethylenediamine. The liquid carriers include water and organic solvents. When water is used as a solvent or a diluent, organic solvents can also be used as additives or antifreeze additives. The suitable organic solvents include aromatic hydrocarbons such as benzene, xylene, and toluene; chlorinated hydrocarbons such as chlorobenzene, chloroethylene, trichloromethane, and dichloromethane; aliphatic hydrocarbons such as petroleum fractions, cyclohexane, and light mineral oil; alcohols such as isopropanol, butanol, glycol, glycerol, and cyclohexanol; ethers and esters thereof; ketones such as acetone, cyclohexanone; dimethylformamide; and N-methylpyrrolidone.

During the preparation of the fungicide composition, the active constituent may be mixed with the liquid carriers and/or solid carriers, surfactants (such as emulsifiers, dispersants, stabilizers, and wetting agents) and other auxiliaries (such as adhesives, defoaming agents, oxidants, and the like) may be added as well.

In a fifth aspect, the present application provides a method of preventing and controlling plant diseases. The method includes: administering an effective dose of the fungicide composition described above to plant diseases to be controlled or to growth media thereof.

Preferably, the effective dose is 10 g per hectare to 1000 g per hectare and preferably 20 g per hectare to 500 g per hectare.

The composition of the present application can be administered to the plant disease and the growth medium thereof in the form of formulations. The compound represented by General Formula I, as the active constituent, is dissolved or dispersed in a carrier or is formulated in a formulation such that the compound is more readily dispersed when it is used as the fungicide. For example, these chemical formulations can be formulated into various liquid formulations, emulsifiable concentrates, suspensions, aqueous suspensions, microemulsions, emulsions, aqueous emulsions, powder, wettable powder, soluble powder, granules, aqueous dispersible granules or capsules.

For certain applications, for example, in agriculture, one or more additional agents, such as fungicides, insecticides, herbicides, plant growth regulators or fertilizers, can be added into the fungicide composition of the present application, so as to obtain additional advantages and effects.

Compared with the existing art, the present application has the following beneficial effects.

The pyrazol-5-ether compound having a structure represented by Formula I in the present application has a remarkable effect on preventing and controlling diseases in agriculture and forestry and especially, exhibits particularly strong control effects against cucumber powdery mildew, cucumber downy mildew, soybean rust, and wheat scab. When the concentration of the pyrazol-5-ether compound is 10 ppm, the control efficacy of the pyrazol-5-ether compound against cucumber powdery mildew is ≥ 90%, and when the concentration of the pyrazol-5-ether compound is 100 ppm, the control efficacy of the pyrazol-5-ether compound against cucumber downy mildew is ≥ 90%. When the concentration of the pyrazol-5-ether compound is 10 ppm, the control efficacy of the pyrazol-5-ether compound against soybean rust is ≥ 90%. When the concentration of the pyrazol-5-ether compound is 100 ppm, the inhibition rate of the pyrazol-5-ether compound against spore germination of wheat scab is ≥ 90%. The preparation method therefor is simple, efficient, and easily scalable for industrial production, indicating broad application prospects.

### DETAILED DESCRIPTION

Technical solutions of the present application will be described below through specific examples. Those skilled in the art are to understand that the examples described herein are used for a better understanding of the present application and are not to be construed as specific limitations to the present application. Unless otherwise specified in the examples and the present application, when the compounds were characterized by proton nuclear magnetic resonance (¹H NMR) spectroscopy, the corresponding samples were dissolved in deuterated dimethylsulfoxide (DMSO-*d₆*) or deuterated chloroform (CDCl₃) and measured by a 400 MHz nuclear magnetic resonance spectrometer to obtain hydrogen spectrum data. Chemical shifts were recorded in parts per million (i.e., δ: ppm). The eluents used for column chromatography purification were prepared according to the volume ratios of petroleum ether (PE) to ethyl acetate (EA).

### Synthesis Example

### Example 1: Preparation of Compound 16

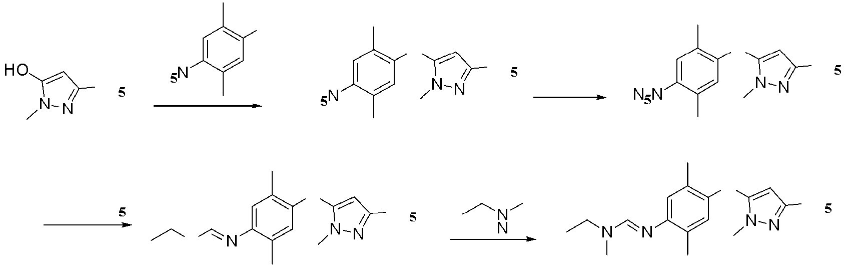

### Step 1: Synthesis of5-(2,5-dimethyl-4-nitrophenoxy)-1-methyl-3-trifluoromethyl-1H-pyrazole

1-chloro-2,5-dimethyl-4-nitrobenzene (2.3 g, 12.5 mmol) and potassium carbonate (2.1 g, 15.1 mmol) were added to 20 mL of dimethylformamide (DMF), and 1-methyl-3-trifluoromethyl-1*H*-pyrazol-5-ol (2.1 g, 12.6 mmol) was added. The resulting mixture was refluxed and reacted. After the thin-layer chromatography (TLC) monitored that the reaction was completed, the reaction solution was then cooled to room temperature. 100 mL of water and 50 mL of ethyl acetate were added. The resulting mixture was extracted. The organic layer was separated, washed with a saturated sodium chloride aqueous solution, dried over anhydrous sodium sulfate, and concentrated under reduced pressure. The residue was purified by column chromatography (using PE:EA = 5:1 as the eluent) to obtain 1.55 g of a pale yellow solid in 39.0% yield.

### Step 2: Synthesis of 2,5-methyl-4-(1-methyl-3-trifluoromethyl-1H-pyrazol-5-yloxy)aniline

5-(2,5-dimethyl-4-nitrophenoxy)-1-methyl-3-trifluoromethyl-1*H*-pyrazole (1.5 g, 4.8 mmol) was added to 30 mL of anhydrous ethanol. Reduced iron powder (1.0 g, 17.6 mmol) was added, and 10 mL of a saturated ammonium chloride aqueous solution was added dropwise. The resulting mixture was refluxed and reacted. After 8 hours, the reaction solution was cooled to room temperature, filtered through Celite, and washed with ethanol. The ethanol was removed by evaporation. 50 mL of water and 50 mL of ethyl acetate were added. The resulting mixture was extracted. The organic layer was separated, washed with a saturated sodium chloride aqueous solution, dried over anhydrous sodium sulfate, and concentrated under reduced pressure. The residue was purified by column chromatography (using PE:EA = 5:1 as the eluent) to obtain 1.24 g of a yellow solid in 91.0% yield.

### Step 3: Synthesis of (E)-N-[4-(1-methyl-3-trifluoromethyl-1H-pyrazol-5-yloxy)-2,5-dimethylphenyl]formamidine ethyl ether

2,5-methyl-4-(1-methyl-3-trifluoromethyl-1*H*-pyrazol-5-yloxy)aniline (1.31 g, 4.6 mmol), triethyl orthoformate (6.8 g, 46.0 mmol), and p-toluenesulfonic acid monohydrate (310 mg, 1.0 mmol) were added to a reaction flask. The resulting mixture was heated to reflux and reacted for 8 hours. The reaction solution was cooled to room temperature and subjected to distillation until no more liquid distilled over to obtain a brown oil. The brown oil was used directly in the next step without further purification.

### Step 4: Synthesis of (E)-N'-[4-(1-methyl-3-trifluoromethyl-1H-pyrazol-5-yloxy)-2,5-dimethylphenyl]-N-ethyl-N-met hylformamidine

(*E*)-*N*-[4-(1-methyl-3-trifluoromethyl-1*H*-pyrazol-5-yloxy)-2,5-dimethylphenyl]forma midine ethyl ether (250 mg, 0.73 mmol) was dissolved in 10 mL of dichloromethane, and methylethylamine (250 mg, 4.2 mmol) was added. The resulting mixture was heated to reflux and reacted. After 2 hours, the reaction was completed. The reaction solution was cooled to room temperature. 20 mL of water was added. The resulting mixture was extracted. The organic layer was separated, washed with a saturated sodium chloride aqueous solution, dried over anhydrous sodium sulfate, and concentrated under reduced pressure. The residue was purified by column chromatography (using PE:EA = 5:1 as the eluent) to obtain 180 mg of Compound 16 as a yellow oil in 69.5% yield.

¹H NMR (DMSO-*d₆*, δ[ppm]) of Compound 16: 7.61 (br S, 1H), 6.90 (s, 1H), 6.72 (s, 1H), 5.74 (s, 1H), 3.81 (s, 3H), 3.33 (br s, 2H), 2.99-2.88 (m, 3H), 2.13 (s, 6H), 1.13 (t, *J=* 6.8 Hz, 3H).

### Example 2: Preparation of Compound 77

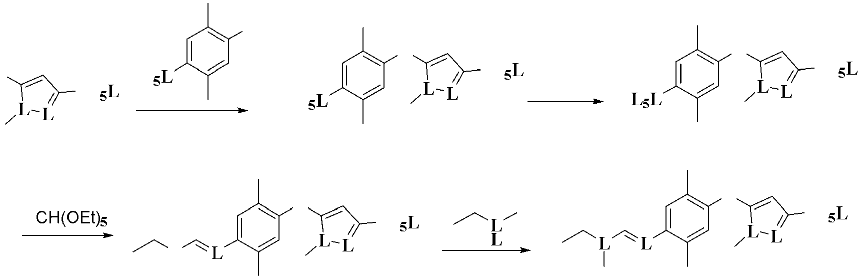

### Step 1: Synthesis of 5-(2,5-dimethyl-4-nitrophenoxy)-1-methyl-3-difluoromethyl-1H-pyrazole

1-chloro-2,5-dimethyl-4-nitrobenzene (3.0 g, 16.2 mmol) and potassium carbonate (4.5 g, 32.3 mmol) were added to 20 mL of DMF, and 1-methyl-3-difluoromethyl-1H-pyrazol-5-ol (2.6 g, 17.8 mmol) was added. The resulting mixture was refluxed and reacted. After the TLC monitored that the reaction was completed, the reaction solution was then cooled to room temperature. 100 mL of water and 50 mL of ethyl acetate were added. The resulting mixture was extracted. The organic layer was separated, washed with a saturated sodium chloride aqueous solution, dried over anhydrous sodium sulfate, and concentrated under reduced pressure. The residue was purified by column chromatography (using PE:EA = 5:1 as the eluent) to obtain 1.3 g of a pale yellow solid in 27.0% yield.

### Step 2: Synthesis of 2,5-methyl-4-(1-methyl-3-difluoromethyl-1H-pyrazol-5-yloxy)aniline

5-(2,5-dimethyl-4-nitrophenoxy)-1-methyl-3-difluoromethyl-1*H*-pyrazole (1.3 g, 4.4 mmol) was added to 30 mL of anhydrous ethanol. Reduced iron powder (490 mg, 8.8 mmol) was added, and 10 mL of a saturated ammonium chloride aqueous solution was added dropwise. The resulting mixture was refluxed and reacted. After 8 hours, the reaction solution was cooled to room temperature, filtered through Celite, and washed with ethanol. The ethanol was removed by evaporation. 50 mL of water and 50 mL of ethyl acetate were added. The resulting mixture was extracted. The organic layer was separated, washed with a saturated sodium chloride aqueous solution, dried over anhydrous sodium sulfate, and concentrated under reduced pressure. The residue was purified by column chromatography (using PE:EA = 5:1 as the eluent) to obtain 830 mg of a pale red oil in 71.2% yield.

### Step 3: Synthesis of (E)-N-[4-(1-methyl-3-difluoromethyl-1H-pyrazol-5-yloxy)-2,5-dimethylphenyl]formamidine ethyl ether

2,5-methyl-4-(1-methyl-3-difluoromethyl-1*H*-pyrazol-5-yloxy)aniline (830 mg, 3.1 mmol), triethyl orthoformate (4.6 g, 31.1 mmol), and *p*-toluenesulfonic acid monohydrate (58.0 mg, 0.3 mmol) were added to a reaction flask. The resulting mixture was heated to reflux and reacted for 8 hours. The reaction solution was cooled to room temperature and subjected to distillation until no more liquid distilled over to obtain a brown oil. The brown oil was used directly in the next step without further purification.

### Step 4: Synthesis of (E)-N'-[4-(1-methyl-3-difluoromethyl-1H-pyrazol-5-yloxy)-2,5-dimethylphenyl]-N-ethyl-N-met hylformamidine

(*E*)-*N*-[4-(1-methyl-3-difluoromethyl-1*H*-pyrazol-5-yloxy)-2,5-dimethylphenyl]forma midine ethyl ether (333.0 mg, 1.0 mmol) was dissolved in 10 mL of dichloromethane, and methylethylamine (365.0 mg, 6.2 mmol) was added. The resulting mixture was heated to reflux and reacted. After 2 hours, the reaction was completed. The reaction solution was cooled to room temperature. 20 mL of water was added. The resulting mixture was extracted. The organic layer was separated, washed with a saturated sodium chloride aqueous solution, dried over anhydrous sodium sulfate, and concentrated under reduced pressure. The residue was purified by column chromatography (using PE:EA = 3:1 as the eluent) to obtain 233.0 mg of Compound 77 as a pale yellow oil in 67.2% yield.

¹H NMR (DMSO-*d₆*, δ[ppm]) of Compound 77: 7.61 (s, 1H), 7.19 (t, *J=* 53.4 Hz, 1H), 6.77 (s, 1H), 6.65 (s, 1H), 5.90 (d, *J* = 1.2 Hz, 1H), 3.75 (s, 3H), 3.32-3.35 (m, 2H), 2.92 (s, 3H), 2.11 (s, 3H), 2.09 (s, 3H), 1.11 (d, *J* = 7.2 Hz, 2H).

### Example 3: Preparation of Compound 99

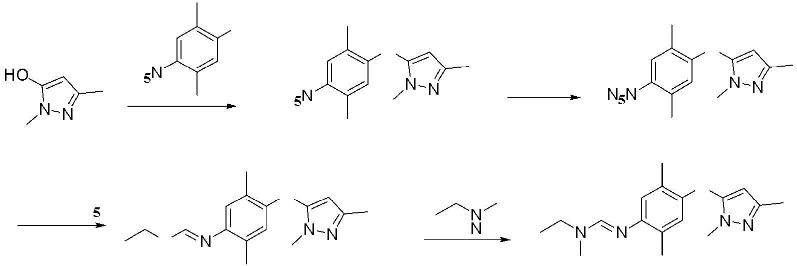

### Step 1: Synthesis of 5-(2,5-dimethyl-4-nitrophenoxy)-1,3-dimethyl-1H-pyrazole

1-chloro-2,5-dimethyl-4-nitrobenzene (3.0 g, 16.2 mmol) and potassium carbonate (4.5 g, 32.3 mmol) were added to 30 mL of DMF, and 1,3-dimethyl-1*H-*pyrazol-5-ol (1.99 g, 17.8 mmol) was added. The resulting mixture was refluxed and reacted. After the TLC monitored that the reaction was completed, the reaction solution was then cooled to room temperature. 100 mL of water and 50 mL of ethyl acetate were added. The resulting mixture was extracted. The organic layer was separated, washed with a saturated sodium chloride aqueous solution, dried over anhydrous sodium sulfate, and concentrated under reduced pressure. The residue was purified by column chromatography (using PE:EA = 10:1 as the eluent) to obtain 1.9 g of a pale tangerine yellow solid in 45.1% yield.

### Step 2: Synthesis of 2,5-methyl-4-(1,3-dimethyl-1H-pyrazol-5-yloxy)aniline

6-(2,5-dimethyl-4-nitrophenoxy)-1,3-dimethyl-1*H*-pyrazole (1.9 g, 7.3 mmol) was added to 30 mL of anhydrous ethanol. Reduced iron powder (814.0 mg, 14.6 mmol) was added, and 10 mL of a saturated ammonium chloride aqueous solution was added dropwise. The resulting mixture was refluxed and reacted. After 8 hours, the reaction solution was cooled to room temperature, filtered through Celite, and washed with ethanol. The ethanol was removed by evaporation. 50 mL of water and 50 mL of ethyl acetate were added. The resulting mixture was extracted. The organic layer was separated, washed with a saturated sodium chloride aqueous solution, dried over anhydrous sodium sulfate, and concentrated under reduced pressure. The residue was purified by column chromatography (using PE:EA = 5:1 as the eluent) to obtain 1.1 g of a yellow solid in 64.7% yield.

### Step 3: Synthesis of (E)-N-[4-(1,3-dimethyl-1H-pyrazol-5-yloxy)-2,5-dimethylphenyl]formamidine ethyl ether

2,5-methyl-4-(1,3-dimethyl-1*H*-pyrazol-5-yloxy)aniline (1.1g, 4.7 mmol), triethyl orthoformate (7.0g, 47.1 mmol), and p-toluenesulfonic acid monohydrate (89.6mg, 0.47 mmol) were added to a reaction flask. The resulting mixture was refluxed and reacted for 8 hours. The reaction solution was cooled to room temperature and subjected to distillation until no more liquid distilled over to obtain a brown oil. The brown oil was used directly in the next step without further purification.

### Step 4: Synthesis of (E)-N'-[4-(1,3-dimethyl-1H-pyrazol-5-yloxy)-2,5-dimethylphenyl]-N-ethyl-N-methylformamidi ne

(*E*)-*N*-[4-(1,3-dimethyl-1*H*-pyrazol-5-yloxy)-2,5-dimethylphenyl]formamidine ethyl ether (337.0 mg, 1.0 mmol) was dissolved in 10 mL of dichloromethane, and methylethylamine (416.0 mg, 6.0 mmol) was added. The resulting mixture was heated to reflux and reacted. After 2 hours, the reaction was completed. The reaction solution was cooled to room temperature. 20 mL of water was added. The resulting mixture was extracted. The organic layer was separated, washed with a saturated sodium chloride aqueous solution, dried over anhydrous sodium sulfate, and concentrated under reduced pressure. The residue was purified by column chromatography (using PE:EA = 3:1 as the eluent) to obtain 273.0 mg of Compound 99 as a pale yellow solid in 77.7% yield.

¹H NMR (DMSO-*d₆*, δ[ppm]) of Compound 99: 7.62 (s, 1H), 6.78 (s, 1H), 6.68 (s, 1H), 5.12 (s, 1H), 3.59 (s, 3H), 3.32 (br s, 2H), 2.92 (s, 3H), 2.12 (s, 3H), 2.11 (s, 3H), 2.02 (s, 3H), 1.12 (t, *J=* 7.2 Hz, 3H).

### Example 4: Preparation of Compound 114

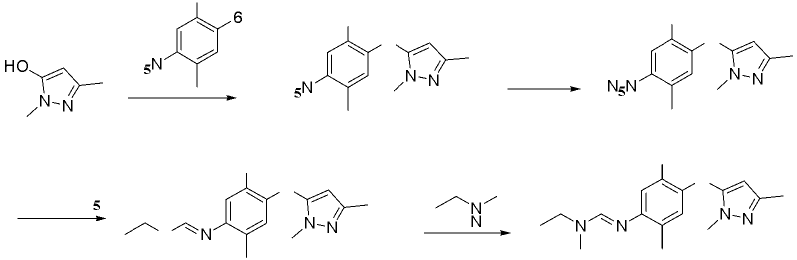

### Step 1: Synthesis of 5-(2-chloro-5-methyl-4-nitrophenoxy)-1,3-dimethyl-1H-pyrazole

1-chloro-2-fluoro-4-methyl-5-nitrobenzene (1.69 g, 8.9 mmol) and potassium carbonate (1.5 g, 10.68 mmol) were added to 20 mL of DMF, and 1,3-dimethyl-1*H* pyrazol-5-ol (1.0 g, 8.9 mmol) was added. The resulting mixture was refluxed and reacted. After 10 hours, the reaction solution was cooled to room temperature. 100 mL of water and 50 mL of ethyl acetate were added. The resulting mixture was extracted. The organic layer was separated, washed with a saturated sodium chloride aqueous solution, dried over anhydrous sodium sulfate, and concentrated under reduced pressure. The residue was purified by column chromatography (using PE:EA = 5:1 as the eluent) to obtain 1.4 g of a yellow solid in 71.1% yield.

### Step 2: Synthesis of 2-methyl-4-(1,3-dimethyl-1H-pyrazol-5-yloxy)-5-chloroaniline

5-(2-chloro-5-methyl-4-nitrophenoxy)-1,3-dimethyl-1*H*-pyrazole (3.24 g, 11.5 mmol) was added to 30 mL of anhydrous ethanol. Reduced iron powder (1.6 g, 28.6 mmol) was added, and 10 mL of a saturated ammonium chloride aqueous solution was added dropwise. The resulting mixture was refluxed and reacted. After 4 hours, the reaction solution was cooled to room temperature, filtered through Celite, and washed with ethanol. The ethanol was removed by evaporation. 50 mL of water and 50 mL of ethyl acetate were added. The resulting mixture was extracted. The organic layer was separated, washed with a saturated sodium chloride aqueous solution, dried over anhydrous sodium sulfate, and concentrated under reduced pressure. The residue was purified by column chromatography (using PE:EA = 2:1 as the eluent) to obtain 1.9 g of a yellow solid in 67.1% yield.

### Step 3: Synthesis of (E)-N-[2-methyl-4-(1,3-dimethyl-1H-pyrazol-5-yloxy)-5-chlorophenyl]formamidine ethyl ether

2-methyl-4-(1,3-dimethyl-1*H*-pyrazol-5-yloxy)-5-chloroaniline (1.9 g, 7.6 mmol), triethyl orthoformate (11.3 g, 76.0 mmol), and p-toluenesulfonic acid monohydrate (144.6 mg, 0.76 mmol) were added to a reaction flask. The resulting mixture was heated to reflux and reacted for 3 hours. The reaction solution was cooled to room temperature and subjected to distillation until no more liquid distilled over to obtain a brown oil. The brown oil was used directly in the next step without further purification.

### Step 4: Synthesis of (E)-N'-[2-methyl-4-(1,3-dimethyl-1H-pyrazol-5-yloxy)-5-chlorophenyl]-N-ethyl-N-methylform amidine

(*E*)-*N*-[2-methyl-4-(1,3-dimethyl-1*H*-pyrazol-5-yloxy)-5-chlorophenyl]formamidine ethyl ether (335.0 mg, 1.1 mmol) was dissolved in 10 mL of dichloromethane, and methylethylamine (389.4 mg, 6.6 mmol) was added. The resulting mixture was heated to reflux and reacted. After the TLC monitored that the reaction was completed, the reaction solution was cooled to room temperature. 20 mL of water was added. The resulting mixture was extracted. The organic layer was separated, washed with a saturated sodium chloride aqueous solution, dried over anhydrous sodium sulfate, and concentrated under reduced pressure. The residue was purified by column chromatography (using PE:EA = 2:1 as the eluent) to obtain 249.0 mg of Compound 114 as a yellow solid in 70.6% yield.

¹H NMR (DMSO-*d₆*, δ[ppm]) of Compound 114: 7.76 (s, 1H), 7.04 (d, *J* = 0.8 Hz, 1H), 7.01 (s, 1H), 5.22 (s, 1H), 3.61 (s, 3H), 3.48-3.34 (m, 2H), 2.92 (s, 3H), 2.14 (s, 3H), 2.03 (s, 3H), 1.13 (d, *J =* 7.2 Hz, 3H).

### Example 5: Preparation of Compound 175

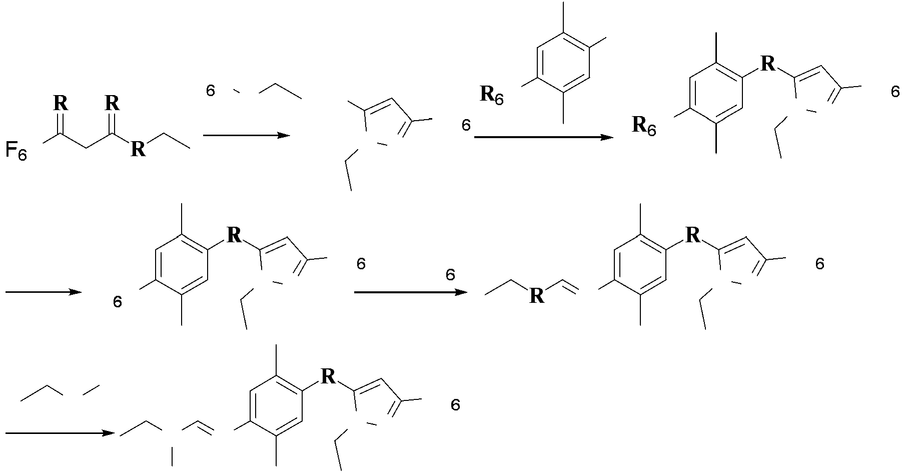

### Step 1: Synthesis of 1-ethyl-3-(trifluoromethyl)-1H-pyrazol-5-ol

Ethyl trifluoroacetoacetate (2.00 g, 10.86 mmol) and ethylhydrazine oxalate (1.96 g, 13.04 mmol) were sequentially added to a flask containing 30 mL of ethanol. The resulting mixture was heated to reflux and reacted. After the TLC monitored that the reaction was completed, the reaction solution was cooled to room temperature. The solvent was removed under reduced pressure. 50 mL of water and 50 mL of ethyl acetate were added. The resulting mixture was extracted. The organic layer was separated, washed with a saturated sodium chloride aqueous solution, dried over anhydrous sodium sulfate, and concentrated under reduced pressure. The residue was used directly in the next step without post-treatment.

### Step 2: Synthesis of 5-(2,5-dimethyl-4-nitrophenoxy)-1-ethyl-3-trifluoromethyl-1H-yrazole

1-chloro-2,5-dimethyl-4-nitrobenzene (1.65 g, 8.9 mmol) and potassium carbonate (1.48 g, 10.68 mmol) were added to 20 mL of DMF, and 1-ethyl-3-(trifluoromethyl)-1*H*-pyrazol-5-ol (1.60 g, 8.9 mmol) was added. The resulting mixture was refluxed and reacted. After 10 hours, the reaction solution was cooled to room temperature. 100 mL of water and 50 mL of ethyl acetate were added. The resulting mixture was extracted. The organic layer was separated, washed with a saturated sodium chloride aqueous solution, dried over anhydrous sodium sulfate, and concentrated under reduced pressure. The residue was purified by column chromatography (using PE:EA = 5:1 as the eluent) to obtain 2.1 g of a yellow solid in 71.7% yield.

### Step 3: Synthesis of 2,5-methyl-4-(1-ethyl-3-trifluoromethyl-1H-pyrazol-5-yloxy)-aniline

5-(2,5-dimethyl-4-nitrophenoxy)-1-ethyl-3-trifluoromethyl-1*H*-pyrazole (3.79 g, 11.5 mmol) was added to 30 mL of anhydrous ethanol. Reduced iron powder (1.6 g, 28.6 mmol) was added, and 10 mL of a saturated ammonium chloride aqueous solution was added dropwise. The resulting mixture was refluxed and reacted. After 4 hours, the reaction solution was cooled to room temperature, filtered through Celite, and washed with ethanol. The ethanol was removed by evaporation. 50 mL of water and 50 mL of ethyl acetate were added. The resulting mixture was extracted. The organic layer was separated, washed with a saturated sodium chloride aqueous solution, dried over anhydrous sodium sulfate, and concentrated under reduced pressure. The residue was purified by column chromatography (using PE:EA = 2:1 as the eluent) to obtain 2.4 g of a brown oily liquid in 69.7% yield.

### Step 4: Synthesis of (E)-N-[2-methyl-4-(1-ethyl-3-trifluoromethyl-1H-pyrazol-5-yloxy)-5-chlorophenyl]formamidin e ethyl ether

2,5-methyl-4-(1-ethyl-3-trifluoromethyl-1*H*-pyrazol-5-yloxy)aniline (2.27 g, 7.6 mmol), triethyl orthoformate (11.26 g, 76.0 mmol), and p-toluenesulfonic acid monohydrate (144.6 mg, 0.76 mmol) were added to a reaction flask. The resulting mixture was heated to reflux and reacted for 3 hours. The reaction solution was cooled to room temperature and subjected to distillation until no more liquid distilled over to obtain a brown oil. The brown oil was used directly in the next step without further purification.

### Step 5: Synthesis of (E)-N'-[4-(1-ethyl-3-trifluoromethyl-1H-pyrazol-5-yloxy)-2,5-dimethylphenyl]-N-ethyl-N-meth ylformamidine

(*E*)-*N*-[2-methyl-4-(1-ethyl-3-trifluoromethyl-1*H*-pyrazol-5-yloxy)-5-chlorophenyl]for mamidine ethyl ether (390.9 mg, 1.1 mmol) was dissolved in 10 mL of dichloromethane, and methylethylamine (389.4 mg, 6.6 mmol) was added. The resulting mixture was heated to reflux and reacted. After the TLC monitored that the reaction was completed, the reaction solution was cooled to room temperature. 20 mL of water was added. The resulting mixture was extracted. The organic layer was separated, washed with a saturated sodium chloride aqueous solution, dried over anhydrous sodium sulfate, and concentrated under reduced pressure. The residue was purified by column chromatography (using PE:EA = 2:1 as the eluent) to obtain 200.0 mg of Compound 175 as a yellow oil in 49.4% yield.

¹H NMR (400 MHz, DMSO-*d₆*) of Compound 175: δ 7.67 (s, 1H), 6.91 (s, 1H), 6.73 (s, 1H), 5.72 (s, 1H), 4.17 (q, *J* = 7.2 Hz, 2H), 3.33-3.41 (m, 2H), 2.92 (s, 3H), 2.13 (s, 6H), 1.39 (t, *J* = 7.2 Hz, 3H), 1.13 (t, *J =* 7.1 Hz, 4H).

In addition to the compounds described in the above examples, compounds in Table 1 can be prepared according to the similar methods as described in Examples 1 to 5. Hereinafter, Table 4 shows the nuclear magnetic resonance data of some compounds prepared by referring to Examples 1 to 5 (samples were dissolved in solvent DMSO-*d₆*, and the solvent of the compounds marked with * was CDCl₃).

**Table 4**

| Compound | ¹H NMR data, 400 MHz δ[ppm] |
|---|---|
| 2 | 7.53 (d, *J* = 2.4 Hz, 1H), 7.09-7.01 (m, 1H), 6.90 (s, 1H), 6.66 (s, 1H), 5.75 (s, 1H), 3.81 (s, 3H), 3.31-3.23 m, 2H), 2.13 (d, *J* = 2.4 Hz, 6H), 1.15 (t, *J* = 7.2 Hz, 3H). |
| 3 | 7.54 (d, *J* = 2.4 Hz, 1H), 7.11-7.03 (m, 1H), 6.90 (s, 1H), 6.66 (br s, 1H), 5.75 (s, 1H), 3.81 (s, 3H), 3.26-3.16 (m, 2H), 2.12 (d, *J* = 4.8 Hz, 6H), 1.62-1.57 (m, 2H), 0.91 (t, *J* = 7.4 Hz, 3H). |
| 15 | 7.62 (s, 1H), 6.91 (s, 1H), 6.72 (s, 1H), 5.75 (s, 1H), 3.80 (s, 3H), 2.96 (d, *J* = 22.4 Hz, 6H), 2.13 (s, 6H). |
| 18 | 7.60 (s, 1H), 6.90 (s, 1H), 6.72 (s, 1H), 5.74 (s, 1H), 3.81 (s, 3H), 3.33 (s, 4H), 2.13 (s, 6H), 1.14 (t, *J* = 7.2 Hz, 6H). |
| 19 | 7.81 (s, 1H), 6.90 (s, 1H), 6.72 (s, 1H), 5.75 (s, 1H), 3.80 (s, 3H), 3.53-3.35 (m, 4H), 2.13 (d, *J* = 1.6 Hz, 6H), 1.87 (br s, 4H). |
| 49 | 7.60 (d, *J* = 33.9 Hz, 1H), 6.71 (s, 1H), 6.42 (s, 1H), 3.67 (s, 3H), 3.33 (m, 2H), 2.90 (s, 3H), 2.25 (s, 3H), 2.07 (s, 3H), 1.74 (d, *J* = 1.3 Hz, 3H), 1.11 (t, *J* = 7.1 Hz, 3H). |
| 63 | 7.74 (s, 1H), 7.19 (d, *J* = 0.9 Hz, 1H), 7.03 (s, 1H), 5.89 (s, 1H), 3.83 (s, 3H), 3.03 (s, 3H), 2.94 (s, 3H), 2.17 (s, 3H). |
| 64 | 7.82-7.76 (m, 1H), 7.20 (d, *J* = 0.9 Hz, 1H), 7.04 (d, *J* = 13.1 Hz, 1H), 5.88 (s, 1H), 3.83 (s, 3H), 3.45 (q, *J* = 7.5 Hz, 2H), 2.97 (s, 3H), 2.17 (s, 3H), 1.19 (t, *J* = 7.5 Hz, 3H). |
| 66 | 7.74 (s, 1H), 7.20 (d, *J* = 0.8 Hz, 1H), 7.06 (s, 1H), 5.88 (s, 1H), 4.08 (q, *J* = 7.1 Hz, 4H), 3.85 (s, 3H), 2.19 (s, 3H), 1.20 (t, *J* = 7.1 Hz, 6H). |
| 67 | 7.49 (d, *J* = 8.0 Hz, 1H), 7.20 (s, 1H), 7.02 (s, 1H), 5.87 (s, 1H), 3.85 (s, 3H), 3.42-3.22 (m, 4H), 2.17 (s, 3H), 1.60 (dh, *J* = 28.7, 7.2 Hz, 4H), 0.88 (dt, *J* = 10.6, 7.2 Hz, 6H). |
| 68 | δ 7.92 (s, 1H), 7.19 (s, 1H), 7.03 (s, 1H), 5.89 (s, 1H), 3.84 (s, 3H), 3.54-3.50 (m, 2H), 2.18 (s, 3H), 1.89-1.85 (m, 6H). |
| 69 | 7.73 (s, 1H), 7.20 (s, 1H), 7.06 (s, 1H), 5.88 (s, 1H), 3.84 (s, 3H), 3.57 (s, 2H), 2.17 (s, 3H), 1.19 (t, *J* = 7.1 Hz, 8H). |
| 70 | 7.71 (s, 1H), 7.21 (s, 1H), 6.99-6.94 (m, 1H), 5.90 (s, 1H), 3.81 (d, *J* = 1.7 Hz, 3H), 3.34 (s, 3H), 2.95 (s, 2H), 2.17 (s, 3H), 1.38-1.14 (m, 3H). |
| 71 | 7.71 (s, 1H), 7.21 (s, 1H), 6.94 (s, 1H), 5.88 (s, 1H), 3.81 (s, 3H), 3.34 (d, *J* = 9.1 Hz, 4H), 2.16 (s, 3H), 1.60 (dh, *J* = 28.5, 7.1 Hz, 4H), 0.87 (d, *J* = 8.2 Hz, 6H). |
| 72 | 7.69 (s, 1H), 7.21 (s, 1H), 6.97 (d, *J* = 0.8 Hz, 1H), 5.88 (s, 1H), 3.81 (s, 3H), 3.44 (q, *J* = 7.2 Hz, 2H), 3.34 (d, *J* = 2.6 Hz, 2H), 2.17 (s, 3H), 1.16 (t, *J* = 7.1 Hz, 6H). |
| 73 | 7.90 (s, 1H), 7.20 (s, 1H), 6.97 (s, 1H), 5.90 (s, 1H), 3.81 (d, *J* = 1.9 Hz, 3H), 3.52 (d, *J* = 6.3 Hz, 4H), 2.16 (s, 3H), 1.88 (s, 4H). |
| 74 | 7.70 (s, 1H), 7.21 (s, 1H), 6.98 (d, *J* = 5.6 Hz, 1H), 5.89 (d, *J* = 4.8 Hz, 1H), 3.81 (d, *J* = 2.0 Hz, 3H), 3.57 (m, 4H), 2.16 (s, 3H), 1.71-1.49 (m, 6H). |
| 75 | 7.76 (s, 1H), 7.21 (s, 1H), 6.97 (d, *J* = 9.5 Hz, 1H), 5.89 (s, 1H), 3.81 (s, 3H), 3.01 (s, 3H), 2.94 (s, 3H), 2.16 (s, 3H). |
| 76 | 7.71 (s, 1H), 7.03 (s, 1H), 6.96 (s, 1H), 5.86 (s, 1H), 3.84 (s, 3H), 3.72 (s, 3H), 3.33 (br s, 2H), 2.89 (s, 3H), 1.12 (t, *J* = 7.2 Hz, 3H). |
| 77 | 7.71 (s, 1H), 7.03 (s, 1H), 6.96 (s, 1H), 5.86 (s, 1H), 3.84 (s, 3H), 3.73 (s, 3H), 3.36 (d, *J* = 7.2 Hz, 2H), 1.21 (d, *J* = 6.7 Hz, 6H), 1.14 (d, *J* = 7.0 Hz, 3H). |
| 79 | 7.59 (s, 1H), 7.19 (t, *J* = 53.4 Hz, 1H), 6.77 (s, 1H), 6.65 (s, 1H), 5.91(t, *J* = 1.2 Hz, 1H), 3.75 (s, 3H), 2.95 (s, 7H), 2.11 (s, 3H), 2.09 (s, 3H). |
| 83 | 7.78 (s, 1H), 7.19 (t, *J* = 53.6 Hz, 1H), 6.77 (s, 1H), 6.65 (s, 1H), 5.91 (t, *J* = 1.2 Hz, 1H), 3.75 (s, 3H), 3.42 (s, 5H), 2.11 (s, 3H), 2.09 (s, 3H), 1.85 (d, *J* = 6.4 Hz, 4H). |
| 101 | 7.59 (s, 1H), 6.78 (s, 1H), 6.67 (s, 1H), 5.12 (s, 1H), 3.59 (br s, 3H), 2.96 (s, 4H), 2.12 (s, 3H), 2.11 (s, 3H), 2.02 (s, 3H). |
| 104 | 7.58 (s, 1H), 6.78 (s, 1H), 6.67 (s, 1H), 5.12 (s, 1H), 3.59 (s, 3H), 3.38 (br s, 4H), 2.12 (s, 3H), 2.11 (s, 3H), 2.02 (s, 3H), 1.14 (t, *J* = 7.1 Hz, 6H). |
| 112 | 7.66 (d, *J* = 3.6 Hz, 1H), 7.20 (s, 1H), 7.04 (s, 1H), 6.92 (s, 1H), 5.23 (s, 1H), 3.61 (s, 3H), 2.79 (d, *J* = 3.6 Hz, 3H), 2.15 (s, 3H), 2.03 (s, 3H). |
| 113 | 7.59 (d, *J* = 3.6 Hz, 1H), 7.26 (s, 1H), 7.04 (s, 1H), 6.91 (s, 1H), 5.23 (s, 1H), 3.61 (s, 3H), 3.30-3.24 (m, 2H), 2.14 (s, 3H), 2.03 (s, 3H), 1.14 (t, *J* = 7.2 Hz, 3H). |
| 115 | 7.71 (s, 1H), 7.04 (d, *J* = 0.8 Hz, 1H), 6.99 (s, 1H), 5.23 (s, 1H), 3.61 (s, 3H), 2.97 (d, *J* = 33.6 Hz, 6H), 2.14 (s, 3H), 2.03 (s, 3H). |
| 116 | 7.70 (s, 1H), 7.04 (d, *J* = 0.8 Hz, 1H), 6.99 (s, 1H), 5.22 (s, 1H), 3.61 (s, 3H), 3.34 (br s, 4H), 2.14 (s, 3H), 2.03 (s, 3H), 1.15 (t, *J* = 7.2 Hz, 6H). |
| 119 | 7.89 (s, 1H), 7.04 (d, *J* = 0.8 Hz, 1H), 6.98 (s, 1H), 5.22 (s, 1H), 3.61 (s, 3H), 3.53-3.35 (m, 4H), 2.15 (s, 3H), 2.03 (s, 3H), 1.90-1.82 (m, 4H). |
| 127 | 7.65 (d, *J* = 3.6 Hz, 1H), 7.31-7.24 (m, 1H), 7.02 (s, 1H), 6.89 (s, 1H), 5.26 (s, 1H), 3.59 (s, 3H), 2.79 (d, *J* = 4.4 Hz, 3H), 2.16 (s, 3H), 2.04 (s, 3H). |
| 128 | 7.57 (d, *J* = 3.6 Hz, 1H), 7.36-7.29 (m, 1H), 7.02 (s, 1H), 6.88 (s, 1H), 5.26 (s, 1H), 3.59 (s, 3H), 3.32-3.23 (m, 2H), 2.16 (s, 3H), 2.04 (s, 3H), 1.15 *(t, J=* 7.2 Hz, 3H). |
| 129 | 7.58 (d, *J* = 3.6 Hz, 1H), 7.34 (s, 1H), 7.01 (s, 1H), 6.87 (s, 1H), 5.25 (s, 1H), 3.58 (s, 3H), 3.22 (d, *J* = 6.4 Hz, 2H), 2.15 (s, 3H), 2.04 (s, 3H), 1.56 (s, 2H), 0.91 (t, *J* = 7.4 Hz, 3H). |
| 130 | 7.69 (s, 1H), 7.02 (s, 1H), 6.93 (s, 1H), 5.25 (s, 1H), 3.59 (s, 3H), 3.02 (s, 3H), 2.94 (s, 3H), 2.16 (s, 3H), 2.04 (s, 3H). |
| 131 | 7.67 (s, 1H), 7.02 (s, 1H), 6.93 (s, 1H), 5.25 (s, 1H), 3.59 (s, 3H), 3.45-3.32 (m, 4H), 2.16 (s, 3H), 2.04 (s, 3H), 1.15 (t, *J* = 7.2 Hz, 6H). |
| 132 | 7.74 (s, 1H), 7.02 (s, 1H), 6.94 (s, 1H), 5.25 (s, 1H), 3.58 (s, 3H), 3.48-3.34 (m, 2H), 2.92 (s, 3H), 2.15 (s, 3H), 2.04 (s, 3H), 1.14 (t, *J* = 6.8 Hz, 3H). |
| 134 | 7.87 (s, 1H), 7.02 (s, 1H), 6.93 (s, 1H), 5.26 (s, 1H), 3.59 (s, 3H), 3.55-3.47(m, 2H), 3.42-3.36 (m, 2H), 2.16 (s, 3H), 2.04 (s, 3H), 1.91-1.83 (m, 4H). |
| 143* | 7.41 (s, 1H), 6.59 (s, 1H), 6.37 (s, 1H), 3.57 (s, 3H), 3.36 (s, 2H), 2.98 (s, 3H), 2.29 (s, 3H), 2.17 (s, 3H), 2.14 (s, 3H), 1.67 (s, 3H), 1.20 (t, *J* = 7.2 Hz, 3H). |
| 145* | 7.63 (s, 1H), 6.62 (s, 1H), 6.37 (s, 1H), 3.57 (s, 3H), 3.54-3.47 (m, 4H), 2.29 (s, 3H), 2.17 (s, 3H), 2.16 (s, 3H), 1.96 (td, *J* = 6.9, 5.5, 3.3 Hz, 4H), 1.67 (s, 3H). |
| 149* | 7.42 (s, 1H), 6.78 (s, 1H), 6.59 (s, 1H), 4.96 (s, 1H), 3.68 (s, 3H), 3.37 (s, 2H), 3.00 (s, 3H), 2.20 (s, 3H), 2.16 (s, 3H), 1.79 (tt, *J= 8.4,* 5.0 Hz, 1H), 1.21 (t, *J=* 7.2 Hz, , 3H), 0.87-0.76 (m2H), 0.66-0.55 (m, 2H). |
| 150* | 7.64 (s, 1H), 6.78 (s, 1H), 6.59 (s, 1H), 4.96 (s, 1H), 3.68 (s, 3H), 3.55-3.43 (m, 4H), 2.21 (s, 3H), 2.16 (s, 3H), 1.99-1.91 (m, 4H), 1.79 (tt, *J* = 8.4, 5.1 Hz, 1H), 0.86-0.78 (m, 2H), 0.70-0.55 (m, 2H). |
| 155 | 7.74-7.69 (m, 2H), 7.66 (s, 1H), 7.37-7.29 (m, 2H), 7.29-7.20 (m, 1H), 6.88 (s, 1H), 6.72 (s, 1H), 5.84 (s, 1H), 3.75 (s, 3H), 3.34 (s, 2H), 3.01-2.83 (m, 3H), 2.17 (s, 3H), 2.14 (s, 3H), 1.13 (t, *J* = 7.2 Hz, 3H). |
| 156 | 7.81 (s, 1H), 7.73-7.66 (m, 2H), 7.38-7.30 (m, 2H), 7.28-7.20 (m, 1H), 6.87 (s, 1H), 6.72 (s, 1H), 5.85 (s, 1H), 3.75 (s, 3H), 3.43 (s, 4H), 2.17 (s, 3H), 2.14 (s, 3H), 1.87 (d, *J* = 6.2 Hz, 4H). |
| 157 | 7.74-7.67 (m, 2H), 7.61 (s, 1H), 7.38-7.30 (m, 2H), 7.30-7.21 (m, 1H), 6.88 (s, 1H), 6.72 (s, 1H), 5.84 (s, 1H), 3.75 (s, 3H), 3.34 (s, 4H), 2.15 (d, *J* = 15.3 Hz, 6H), 1.28-1.11 (m, 6H). |
| 158 | 7.75-7.67 (m, 2H), 7.60 (s, 1H), 7.38-7.30 (m, 2H), 7.30-7.21 (m, 1H), 6.88 (s, 1H), 6.73 (s, 1H), 5.84 (s, 1H), 3.75 (s, 3H), 3.35 (s, 2H), 2.36-2.25 (m, 2H), 2.15 (d, *J* = 18.9 Hz, 6H), 1.57-1.42 (m, 6H). |
| 162 | 7.71 (s, 1H), 7.00 (d, *J* = 8.6 Hz, 1H), 6.88 (d, *J* = 2.7 Hz, 1H), 6.78 (dd, *J* = 8.5, 2.7 Hz, 1H), 5.77 (s, 1H), 3.82 (s, 3H), 3.67-3.14 (m, 4H), 2.18 (s, 3H), 1.50 (q, *J* = 6.0, 4.9 Hz, 6H). |
| 163 | 7.93 (s, 1H), 6.99 (d, *J* = 8.6 Hz, 1H), 6.88 (d, *J* = 2.6 Hz, 1H), 6.78 (dd, *J* = 8.5, 2.7 Hz, 1H), 5.78 (s, 1H), 3.81 (s, 3H), 3.63-3.39 (m, 4H), 2.18 (s, 3H), 1.86 (m, 4H). |
| 164 | 7.93 (s, 1H)), 6.99 (s, 1H), 6.90-6.84 (m, 1H), 6.80-6.75 (m, 1H), 5.77 (s, 1H), 3.82 (s, 3H), 2.18 (s, 3H), 1.75-1.44 (m, 2H), 1.25 (t, *J* = 10.7 Hz, 3H), 0.83 (t, *J* = 7.4 Hz, 3H). |
| 165 | 7.73 (s, 1H), 6.99 (d, *J* = 8.5 Hz, 1H), 6.88 (d, *J* = 2.7 Hz, 1H), 6.78 (dd, *J* = 8.5, 2.7 Hz, 1H), 5.76 (s, 1H), 3.81 (s, 3H), 3.40 (s, 4H), 2.17 (s, 3H), 1.22-1.09 (m, 6H). |
| 166 | 7.80-7.64 (m, 1H), 7.00 (d, *J* = 8.6 Hz, 1H), 6.88 (d, *J* = 2.8 Hz, 1H), 6.78 (dd, *J* = 8.6, 2.7 Hz, 1H), 5.77 (s, 1H), 3.82 (s, 3H), 3.36 (d, *J* = 12.5 Hz, 2H), 2.94 (d, *J* = 30.9 Hz, 3H), 2.18 (s, 3H), 1.13 (t, *J* = 6.9 Hz, 3H). |
| 167 | 7.73 (s, 1H), 7.00 (d, *J* = 8.6 Hz, 1H), 6.87 (d, *J* = 2.6 Hz, 1H), 6.78 (dd, *J* = 8.6, 2.7 Hz, 1H), 5.78 (s, 1H), 3.81 (s, 3H), 2.95 (d, *J* = 37.0 Hz, 6H), 2.18 (s, 3H). |
| 168 | 7.73 (s, 1H), 6.99 (d, *J* = 8.5 Hz, 1H), 6.88 (d, *J* = 2.7 Hz, 1H), 6.78 (dd, *J* = 8.5, 2.7 Hz, 1H), 5.76 (s, 1H), 3.81 (s, 3H), 3.40 (s, 4H), 2.17 (s, 3H), 1.22-1.09 (m, 6H). |
| 169 | 7.68 (s, 1H), 7.00 (s, 1H), 6.62 (s, 1H), 5.68 (s, 1H), 3.78 (d, *J* = 14.6 Hz, 6H), 3.33 (s, 4H), |
| | 2.10 (s, 3H), 1.15 (t, *J* = 7.1 Hz, 6H). |
| 170 | 7.69 (d, *J* = 42.5 Hz, 1H), 7.00 (s, 1H), 6.62 (s, 1H), 5.68 (s, 1H), 3.77 (d, *J* = 15.4 Hz, 6H), 2.96 (d, *J* = 26.7 Hz, 3H), 2.10 (s, 3H), 1.23 (s, 2H), 1.14 (t, *J* = 7.0 Hz, 3H). |
| 171 | 7.68 (s, 1H), 7.01 (s, 1H), 6.63 (s, 1H), 5.68 (s, 1H), 3.77 (d, *J* = 14.7 Hz, 6H), 3.53 (s, 2H), 2.09 (s, 3H), 1.63 (t, *J* = 5.8 Hz, 2H), 1.55-1.48 (m, 5H), 1.24 (s, 1H). |
| 172 | 7.82-7.76 (m, 2H), 7.72 (s, 1H), 7.59 (dd, *J* = 8.7, 7.1 Hz, 2H), 7.56-7.42 (m, 1H), 7.11 (d, *J* = 8.6 Hz, 1H), 6.89 (d, *J* = 2.7 Hz, 1H), 6.80 (dd, *J* = 8.5, 2.7 Hz, 1H), 5.95 (s, 1H), 4.08 (q, *J* = 7.0 Hz, 2H), 2.93 (d, *J* = 7.9 Hz, 3H), 2.15 (s, 3H), 1.20 (t, *J* = 7.1 Hz, 3H). |
| 173 | 7.82-7.76 (m, 2H), 7.72 (s, 1H), 7.59 (dd, *J* = 8.7, 7.1 Hz, 2H), 7.56-7.42 (m, 1H), 7.11 (d, *J* = 8.6 Hz, 1H), 6.89 (d, *J* = 2.7 Hz, 1H), 6.80 (dd, *J* = 8.5, 2.7 Hz, 1H), 5.95 (s, 1H), 3.53-3.43 (m, 4H), 2.15 (s, 3H), 1.86 (m, 4H). |
| 174 | 7.82-7.76 (m, 2H), 7.72 (s, 1H), 7.59 (dd, *J* = 8.7, 7.1 Hz, 2H), 7.56-7.42 (m, 1H), 7.11 (d, *J* = 8.6 Hz, 1H), 6.89 (d, *J* = 2.7 Hz, 1H), 6.80 (dd, *J* = 8.5, 2.7 Hz, 1H), 5.95 (s, 1H), 3.44 (d, *J* = 54.0 Hz, 4H), 2.15 (s, 3H), 1.69-1.44 (m, 6H). |
| 175 | 7.67 (s, 1H), 6.91 (s, 1H), 6.73 (s, 1H), 5.72 (s, 1H), 4.17 (q, *J* = 7.2 Hz, 2H), 3.33-3.41 (m, 2H), 2.92 (s, 3H), 2.13 (s, 6H), 1.39 (t, *J* = 7.2 Hz, 3H), 1.13 (t, *J* = 7.1 Hz, 4H). |
| 176 | 7.82 (s, 1H), 6.91 (s, 1H), 6.73 (s, 1H), 5.72 (s, 1H), 4.17 (q, *J* = 7.2 Hz, 2H), 3.53-3.45 (m, 4H), 2.14 (d, *J* = 4.2 Hz, 6H), 1.87 (s, 4H), 1.39 (t, *J* = 7.2 Hz, 3H). |
| 177 | 7.65 (s, 1H), 6.92 (s, 1H), 6.78 (s, 1H), 5.73 (s, 1H), 4.17 (q, *J* = 7.2 Hz, 2H), 3.72-3.40 (m, 4H), 2.14 (d, *J* = 2.1 Hz, 6H), 1.38 (t, *J=* 7.2 Hz, 3H), 1.19 (t, *J* = 7.1 Hz, 6H). |
| 178 | 7.62 (s, 1H), 6.91 (s, 1H), 6.72 (s, 1H), 5.72 (s, 1H), 4.17 (q, *J* = 7.2 Hz, 2H), 3.07-2.84 (m, 6H), 2.13 (s, 6H), 1.38 *(t, J=* 7.2 Hz, 3H). |
| 179 | 7.61 (s, 1H), 6.91 (s, 1H), 6.73 (s, 1H), 5.71 (s, 1H), 4.17 (q, *J* = 7.2 Hz, 2H), 3.34 (s, 4H), 2.13 (s, 6H), 1.39 (t, *J* = 7.2 Hz, 3H), 1.15 (t, *J* = 7.1 Hz, 6H). |
| 180 | δ 7.58 (s, 1H), 6.91 (s, 1H), 6.73 (s, 1H), 5.70 (s, 1H), 4.17 (q, *J* = 7.2 Hz, 2H), 3.34 (s, 2H), 3.22 (ddd, *J* = 13.1, 11.5, 2.9 Hz, 1H), 2.13 (d, *J* = 2.2 Hz, 6H), 1.67-1.58 (m, 3H), 1.39 (t, *J* = 7.2 Hz, 3H), 1.28-1.19 (m, 6H). |
| 181 | δ 7.68 (s, 1H), 6.93 (s, 1H), 6.79 (s, 1H), 5.73 (s, 1H), 4.17 (q, *J* = 7.2 Hz, 2H), 3.58 (d, 4H), 2.14 (d, *J* = 2.8 Hz, 6H), 2.06-1.94 (m, 4H), 1.39 (t, *J* = 7.2 Hz, 3H). |
| 182 | δ 7.64 (s, 1H), 6.91 (s, 1H), 6.72 (s, 1H), 5.71 (s, 1H), 4.17 (q, *J* = 7.2 Hz, 2H), 3.52 (dd, *J* = 6.8, 5.1 Hz, 2H), 3.43 (t, *J* = 5.9 Hz, 2H), 2.13 (s, 6H), 1.75 (q, *J* = 6.2, 5.8 Hz, 2H), 1.68 (q, *J=* 5.7 Hz, 2H), 1.54 (h, *J* = 2.3 Hz, 4H), 1.39 (t, *J* = 7.3 Hz, 3H). |
| 183 | 7.46 (s, 1H), 7.13-6.99 (m, 1H), 6.90 (s, 1H), 6.66 (s, 1H), 5.72 (s, 1H), 4.17 (q, *J* = 7.2 Hz, 2H), 3.93-3.55 (m, 1H), 2.13 (s, 6H), 1.54-1.50 (m, 4H), 1.38 (td, *J* = 7.4, 2.5 Hz, 4H). |
| 184 | 7.92 (s, 1H), 7.20 (d, *J* = 0.8 Hz, 1H), 7.04 (s, 1H), 5.86 (s, 1H), 4.19 (q, *J* = 7.2 Hz, 2H), 3.52 (t, *J* = 6.4 Hz, 2H), 3.38 (d, *J* = 7.4 Hz, 2H), 2.18 (s, 3H), 1.86 (d, *J* = 11.0 Hz, 4H), 1.40 (t, *J* = 7.2 Hz, 3H). |
| 185 | 7.73 (s, 1H), 7.20 (d, *J* = 0.9 Hz, 1H), 7.05 (s, 1H), 5.85 (s, 1H), 4.19 (q, *J* = 7.2 Hz, 2H), 3.56 (s, 2H), 3.37 (s, 2H), 2.18 (s, 3H), 1.53 (s, 6H), 1.19 (t, *J=* 7.1 Hz, 3H). |
| 186 | 7.79 (s, 1H), 7.20 (d, *J* = 0.9 Hz, 1H), 7.04 (d, *J* = 13.1 Hz, 1H), 5.85 (s, 1H), 4.19 (q, *J* = 7.2 Hz, 2H), 3.34 (s, 3H), 2.93 (m, 2H), 2.17 (s, 3H), 1.40 (t, *J* = 7.2 Hz, 3H), 1.13 (q, *J* = 7.1 Hz, 3H). |
| 187 | 7.74 (s, 1H), 7.20 (d, *J* = 0.9 Hz, 1H), 7.04 (s, 1H), 5.86 (s, 1H), 4.19 (q, *J* = 7.2 Hz, 2H), 3.03 (s, 3H), 2.94 (s, 3H), 2.17 (s, 3H), 1.40 (t, *J* = 7.2 Hz, 3H). |
| 188 | 7.73 (s, 1H), 7.20 (d, *J* = 0.9 Hz, 1H), 7.05 (s, 1H), 5.85 (s, 1H), 4.19 (q, *J* = 7.2 Hz, 2H), 3.44 (q, *J* = 7.0 Hz, 2H), 3.35 (s, 2H), 2.17 (s, 3H), 1.40 (t, *J* = 7.2 Hz, 3H), 1.15 (t, J= 7.1 Hz, 6H). |
| 189 | 7.75 (s, 1H), 7.20 (d, *J* = 0.9 Hz, 1H), 7.01 (s, 1H), 5.85 (s, 1H), 4.19 (q, *J* = 7.2 Hz, 2H), 3.35 (d, *J* = 7.4 Hz, 2H), 3.26 (t, *J* = 7.2 Hz, 2H), 2.16 (s, 3H), 1.60 (dh, *J* = 27.3, 7.4 Hz, 4H), 1.40 (t, *J* = 7.2 Hz, 3H), 0.87 (dt, J= 10.8, 7.2 Hz, 6H). |
| 190 | 7.70 (s, 1H), 7.20 (d, *J* = 0.9 Hz, 1H), 7.05 (s, 1H), 5.84 (s, 1H), 4.18 (q, *J* = 7.2 Hz, 2H), 3.34 (m, 2H), 3.24 (td, *J* = 13.2, 12.4, 2.8 Hz, 1H), 2.16 (s, 3H), 1.66-1.60 (m, 3H), 1.39 (t, *J=* 7.2 Hz, 3H), 1.31-1.11 (m, 6H). |
| 191 | 7.79 (s, 1H), 7.23 (d, *J* = 0.9 Hz, 1H), 7.13 (s, 1H), 5.87 (s, 1H), 4.19 (q, *J* = 7.2 Hz, 2H), 3.61 (d, *J* = 60.4 Hz, 4H), 2.17 (s, 3H), 2.07-1.97 (m, 4H), 1.40 (t, *J* = 7.3 Hz, 3H). |
| 192 | 7.76 (s, 1H), 7.20 (d, *J* = 0.9 Hz, 1H), 7.05 (s, 1H), 5.84 (s, 1H), 4.19 (q, *J* = 7.2 Hz, 2H), 3.57-3.50 (m, 2H), 3.46 (t, *J* = 5.9 Hz, 2H), 2.17 (s, 3H), 1.56-1.50 (m, 8H), 1.40 (t, *J* = 7.3 Hz, 3H). |
| 193 | 7.70 (s, 1H), 7.21 (s, 1H), 6.98 (d, *J* = 0.9 Hz, 1H), 5.86 (s, 1H), 4.17 (q, *J* = 7.2 Hz, 2H), 3.45 (q, *J* = 7.0 Hz, 2H), 3.35 (M, 2H), 2.16 (s, 3H), 1.39 (t, *J* = 7.3 Hz, 3H), 1.16 (t, *J* = 7.1 Hz, 6H). |
| 194 | 7.72 (s, 1H), 7.21 (s, 1H), 6.98 (d, *J* = 0.8 Hz, 1H), 5.88 (s, 1H), 4.18 (q, *J* = 7.2 Hz, 2H), 3.04 (s, 3H), 2.96 (s, 3H), 2.17 (s, 3H), 1.39 (t, *J* = 7.2 Hz, 3H). |
| 195 | 7.71 (s, 1H), 7.21 (s, 1H), 6.97-6.93 (m, 1H), 5.86 (s, 1H), 4.17 (q, *J* = 7.2 Hz, 2H), 3.35 (d, *J* = 7.8 Hz, 2H), 3.26 (t, *J* = 7.2 Hz, 2H), 2.16 (s, 3H), 1.61 (dq, *J* = 30.4, 7.6 Hz, 4H), 1.39 (t, *J* = 7.2 Hz, 3H), 0.93-0.82 (m, 6H). |
| 196 | 7.71 (d, *J* = 45.5 Hz, 1H), 7.21 (s, 1H), 6.98 (d, *J* = 10.0 Hz, 1H), 5.87 (s, 1H), 4.17 (q, *J*= 7.2 Hz, 2H), 3.35 (s, 3H), 2.94 (s, 2H), 2.16 (s, 3H), 1.38 (t, *J=* 7.2 Hz, 3H), 1.15 (t, *J* = 7.0 Hz, 3H). |
| 197 | 7.90 (s, 1H), 7.20 (s, 1H), 6.97 (d, *J* = 0.8 Hz, 1H), 5.88 (s, 1H), 4.17 (q, *J* = 7.2 Hz, 2H), 3.53 (t, *J* = 6.4 Hz, 2H), 3.35 (s, 2H), 2.16 (s, 3H), 1.91-1.84 (m, 4H), 1.38 (t, *J* = 7.2 Hz, 3H). |
| 199 | 7.60 (s, 1H), 6.90 (s, 1H), 6.73 (s, 1H), 5.69 (s, 1H), 4.10 (t, *J* = 6.9 Hz, 2H), 3.34 (s, 4H), 2.12 (s, 6H), 1.82 (h, *J* = 7.2 Hz, 2H), 1.49 (dq, *J* = 22.6, 55 Hz, 6H), 0.89 (t, *J* = 7.4 Hz, 3H). |
| 200 | 7.61 (s, 1H), 6.90 (s, 1H), 6.73 (s, 1H), 5.69 (s, 1H), 4.10 (t, *J=* 6.9 Hz, 2H), 3.42-3.11 (m, 4H), 2.13 (d, *J* = 2.1 Hz, 6H), 1.82 (h, *J* = 7.2 Hz, 2H), 1.14 (t, *J* = 7.0 Hz, 6H), 0.89 (t, *J=* 7.4 Hz, 3H). |
| 201 | 7.69-7.65 (m, 1H), 6.90 (s, 1H), 6.73 (s, 1H), 5.70 (s, 1H), 4.10 (t, *J=* 6.9 Hz, 2H), 3.33 (d, *J* = 9.0 Hz, 3H), 2.92 (s, 2H), 2.13 (d, *J* = 2.4 Hz, 6H), 1.82 (h, *J* = 7.2 Hz, 2H), 1.13 (t, *J* = 7.1 Hz, 3H), 0.89 (t, *J* = 7.4 Hz, 3H). |
| 202 | 7.79 (s, 1H), 7.19 (s, 1H), 7.04 (d, *J* = 13.3 Hz, 1H), 5.85 (s, 1H), 4.12 (t, *J* = 7.0 Hz, 2H), 3.35 (d, *J* = 10.8 Hz, 2H), 2.97 (d, *J* = 29.8 Hz, 3H), 2.17 (s, 3H), 1.83 (h, *J* = 7.2 Hz, 2H), 1.13 (q, *J* = 7.2 Hz, 3H), 0.89 (t, *J* = 7.4 Hz, 3H). |
| 203 | 7.73 (s, 1H), 7.19 (s, 1H), 7.05 (s, 1H), 5.84 (s, 1H), 4.12 (t, *J=* 7.0 Hz, 2H), 3.51-3.27 (m, 4H), 2.17 (s, 3H), 1.83 (h, *J* = 7.3 Hz, 2H), 1.15 (t, *J* = 7.1 Hz, 6H), 0.89 (t, *J* = 7.4 Hz, 3H). |
| 204 | 7.73 (s, 1H), 7.19 (s, 1H), 7.05 (s, 1H), 5.84 (s, 1H), 4.12 (t, *J* = 7.0 Hz, 2H), 3.36 (d, *J* = 14.3 Hz, 4H), 2.16 (s, 3H), 1.83 (h, *J* = 7.2 Hz, 2H), 1.53 (s, 6H), 0.89 (t, *J* = 7.4 Hz, 3H). |
| 205 | 7.79 (s, 1H), 7.19 (s, 1H), 7.01 (s, 1H), 5.83 (s, 1H), 4.12 (t, *J* = 7.0 Hz, 2H), 3.33 (s, 2H), 2.17 (s, 3H), 1.85-1.77 (m, 2H), 1.29-1.24 (m, 12H), 0.90 (t, *J* = 7.4 Hz, 3H). |
| 206 | 7.75 (s, 1H), 7.19 (d, *J* = 3.3 Hz, 1H), 7.01 (s, 1H), 5.84 (s, 1H), 4.12 (t, *J* = 7.0 Hz, 3H), 3.34 (s, 4H), 1.85-1.77 (m, 3H), 1.62 (ddt, *J* = 30.8, 14.6, 7.2 Hz, 4H), 0.89 (d, *J* = 1.3 Hz, 2H), 0.88-0.79 (m, 6H). |
| 207 | 7.67 (s, 1H), 6.91 (s, 1H), 6.73 (s, 1H), 5.68 (s, 1H), 4.71 (hept, *J* = 6.6 Hz, 1H), 3.34 (s, 3H), 2.94-2.90 (m, 2H), 2.13 (s, 6H), 1.44 (d, *J* = 6.6 Hz, 6H), 1.13 (t, *J* = 7.1 Hz, 3H) |
| 208 | 7.61 (s, 1H), 6.91 (s, 1H), 6.73 (s, 1H), 5.68 (s, 1H), 4.71 (hept, *J* = 6.6 Hz, 1H), 3.34 (s, 4H), 2.13 (s, 6H), 1.44 (d, *J* = 6.6 Hz, 6H), 1.15 (t, *J* = 7.1 Hz, 6H). |
| 209 | 7.60 (s, 1H), 6.90 (s, 1H), 6.73 (s, 1H), 5.67 (s, 1H), 4.71 (hept, *J* = 6.7 Hz, 1H), 3.34 (s, 4H), 2.13 (d, *J* = 1.9 Hz, 6H), 1.65-1.58 (m, 6H), 1.44 (s, 6H). |
| 210 | 7.62 (s, 1H), 6.91 (s, 1H), 6.70 (s, 1H), 5.68 (s, 1H), 3.31 (s, 1H), 3.24 (s, 4H), 2.12 (d, *J* = 3.4 Hz, 6H), 1.44 (d, *J* = 6.7 Hz, 6H), 1.37-1.20 (m, 4H), 0.86 (d, *J* = 7.7 Hz, 6H). |
| 212 | 7.79 (s, 1H), 7.23-7.18 (m, 1H), 7.04 (d, *J* = 13.4 Hz, 1H), 5.81 (s, 1H), 4.73 (hept, *J =* 6.7 Hz, 1H), 3.34 (s, 2H), 2.93 (s, 3H), 2.17 (s, 3H), 1.45 (d, *J* = 6.7 Hz, 6H), 1.13 (q, *J* = 7.2 Hz, 3H). |
| 213 | 7.79 (s, 1H), 7.23-7.18 (m, 1H), 7.04 (d, *J* = 13.4 Hz, 1H), 5.81 (s, 1H), 4.73 (hept, *J* = 6.7 Hz, 1H), 3.34 (s, 2H), 2.93 (s, 2H), 2.17 (s, 3H), 1.45 (d, *J* = 6.7 Hz, 6H), 1.13 (q, *J* = 7.2 Hz, 6H). |
| 214 | 7.73 (s, 1H), 7.20 (s, 1H), 7.05 (s, 1H), 5.81 (s, 1H), 4.72 (hept, *J* = 6.7 Hz, 1H), 3.56 (s, 4H), 2.16 (s, 3H), 1.53 (m, 6H), 1.45 (d, *J =* 6.7 Hz, 6H). |
| 215 | 7.73-7.53 (m, 1H), 6.90 (s, 1H), 6.74 (s, 1H), 5.61 (s, 1H), 3.35 (d, *J =* 7.1 Hz, 2H), 2.93 (s, 3H), 2.14 (s, 6H), 1.66 (s, 9H), 1.14 (t, *J* = 7.1 Hz, 3H). |
| 216 | 7.61 (s, 1H), 6.90 (s, 1H), 6.74 (s, 1H), 5.60 (s, 1H), 3.37 (d, *J =* 27.2 Hz, 4H), 2.13 (s, 6H), 1.65 (s, 9H), 1.15 (t, *J* = 7.1 Hz, 6H). |
| 217 | 7.60 (s, 1H), 6.90 (s, 1H), 6.74 (s, 1H), 5.60 (s, 1H), 3.34 (m, 4H), 2.13 (d, *J* = 3.3 Hz, 6H), 1.65 (s, 9H), 1.53 (q, *J* = 4.8, 3.7 Hz, 6H). |
| 218 | 7.62 (s, 1H), 6.90 (s, 1H), 6.71 (s, 1H), 5.61 (s, 1H), 3.34 (s, 2H), 3.24 (s, 2H), 2.12 (d, *J* = 4.8 Hz, 6H), 1.65 (s, 9H), 1.58 (s, 4H), 0.87 (s, 6H). |
| 219 | 7.68 (s, 1H), 6.90 (s, 1H), 6.71 (s, 1H), 5.61 (s, 1H), 3.35 (s, 2H), 2.13 (d, *J* = 3.8 Hz, 6H), 1.65 (s, 9H), 1.25 (d, *J* = 6.8 Hz, 12H). |
| 220 | 7.80 (s, 1H), 7.18 (s, 1H), 7.05 (d, *J* = 13.4 Hz, 1H), 5.76 (s, 1H), 3.36 (d, *J* = 8.2 Hz, 2H), 2.98 (d, *J* = 29.2 Hz, 3H), 2.18 (s, 3H), 1.66 (s, 9H), 1.14 (q, *J* = 7.1 Hz, 3H). |
| 221 | 7.73 (s, 1H), 7.18 (s, 1H), 7.06 (s, 1H), 5.76 (s, 1H), 3.34 (s, 4H), 2.17 (s, 3H), 1.65 (s, 9H), 1.15 (t, *J* = 7.1 Hz, 6H). |
| 222 | 7.73 (s, 1H), 7.20-7.15 (m, 1H), 7.06 (s, 1H), 5.75 (s, 1H), 3.33 (s, 4H), 2.16 (s, 3H), 1.65 (s, 9H), 1.51 (d, *J* = 11.4 Hz, 4H), 1.24 (d, *J* = 3.1 Hz, 2H). |
| 223 | 7.79 (s, 1H), 7.17 (d, *J* = 0.9 Hz, 1H), 7.02 (s, 1H), 5.75 (s, 1H), 3.35 (s, 2H), 3.26 (t, *J* = 7.3 Hz, 2H), 2.16 (s, 3H), 1.65 (s, 9H), 1.56-1.50 (m, 4H), 0.87 (dt, *J* = 11.1, 7.3 Hz, 6H). |
| 224 | 7.79 (s, 1H), 7.17 (d, *J* = 0.9 Hz, 1H), 7.02 (s, 1H), 5.75 (s, 1H), 3.33 (s, 2H), 2.17 (s, 3H), 1.66 (s, 9H), 1.27 (d, *J* = 6.7 Hz, 12H). |
| 225* | 7.43 (d, J = 37.2 Hz, 1H), 7.02 (s, 1H), 6.84 (s, 1H), 5.58 (s, 1H), 4.77 (q, *J* = 8.2 Hz, 2H), 3.61-3.27 (m, 2H), 3.03 (s, 3H), 2.25 (s, 3H), 1.24 (t, *J* = 7.3 Hz, 3H). |
| 226 | 7.74 (s, 1H), 7.22 (d, J = 0.8 Hz, 1H), 7.07 (s, 1H), 5.95 (s, 1H), 5.25 (q, *J* = 9.0 Hz, 2H), 3.52-3.23 (m, 4H), 2.18 (s, 3H), 1.16 (t, *J* = 7.1 Hz, 6H). |
| 227 | 7.77 (s, 1H), 7.22 (d, *J* = 1.4 Hz, 1H), 7.04 (s, 1H), 5.95 (s, 1H), 5.27-5.24 (m, 2H), 3.31 (d, *J=* 32.4 Hz, 4H), 2.17 (s, 3H), 1.60 (dh, *J* = 28.6, 7.4 Hz, 4H), 0.88 (dt, *J* = 10.6, 7.2 Hz, 6H). |
| 228 | 7.74 (s, 1H), 7.22 (d, *J* = 0.8 Hz, 1H), 7.07 (s, 1H), 5.94 (s, 1H), 5.25 (q, *J* = 8.9 Hz, 2H), 3.36 (d, *J* = 15.4 Hz, 4H), 2.17 (s, 3H), 1.53 (s, 6H). |
| 229 | 7.80 (s, 1H), 7.22 (s, 1H), 7.04 (s, 1H), 5.93 (s, 1H), 5.26 (q, *J* = 8.9 Hz, 2H), 4.41 (p, *J* = 6.8 Hz, 1H), 3.72 (p, *J* = 6.8 Hz, 1H), 2.18 (s, 3H), 1.27 (t, *J* = 5.9 Hz, 12H). |
| 230 | 7.79 (s, 1H), 7.21 (d, *J* = 0.9 Hz, 1H), 7.04 (d, *J* = 12.7 Hz, 1H), 5.82 (s, 1H), 3.65 (tt, *J* = 7.3, 3.8 Hz, 1H), 3.33 (s, 3H), 2.93 (s, 2H), 2.17 (s, 3H), 1.18-1.14 (m, 3H), 1.13-1.10 (m, 2H), 1.09-1.04 (m, 2H). |
| 231 | 7.72 (s, 1H), 7.20 (d, *J* = 0.9 Hz, 1H), 7.05 (s, 1H), 5.82 (s, 1H), 3.65 (tt, *J* = 7.3, 3.8 Hz, 1H), ), 3.33 (s, 4H), 2.17 (s, 3H), 1.16 (d, *J* = 7.1 Hz, 6H), 1.14-1.11 (m, 2H), 1.07 (tdd, *J* = 6.7, 5.5, 2.6 Hz, 2H). |
| 232 | 7.79 (s, 1H), 7.21 (s, 1H), 7.01 (s, 1H), 5.81 (s, 1H), 3.65 (dq, *J* = 7.3, 3.7 Hz, 1H), 3.33 (s, 2H), 2.17 (s, 3H), 1.28-1.24 (m, 12H), 1.14 (tt, *J* = 3.9, 2.2 Hz, 2H), 1.09-1.04 (m, 2H). |
| 233 | 7.75 (s, 1H), 7.20 (d, *J* = 2.9 Hz, 1H), 7.02 (s, 1H), 5.82 (s, 1H), 3.65 (tt, *J* = 7.3, 3.8 Hz, 1H), 3.35 (d, *J* = 8.1 Hz, 4H), 2.16 (s, 3H), 1.60 (dq, *J* = 22.8, 7.3 Hz, 4H), 1.17-1.11 (m, 2H), 1.10-1.03 (m, 2H), 0.87 (dt, *J* = 10.7, 7.3 Hz, 6H). |
| 234 | 7.73 (s, 1H), 7.20 (d, *J* = 0.9 Hz, 1H), 7.05 (s, 1H), 5.82 (s, 1H), 3.65 (tt, *J* = 7.3, 3.8 Hz, 1H), 3.34 (s, 4H), 2.16 (s, 3H), 1.53 (s, 6H), 1.18-1.10 (m, 2H), 1.07 (tdd, *J* = 6.6, 5.4, 2.5 Hz, 2H). |
| 235 | 7.58 (s, 1H), 6.72 (s, 1H), 6.42 (s, 1H), 3.67 (s, 3H), 3.41 (s, 4H), 2.25 (s, 3H), 2.06 (s, 3H), 1.74 (d, *J* = 1.2 Hz, 3H), 1.62 (dt, *J* = 11.1, 5.2 Hz, 2H), 1.51 (q, *J* = 5.9 Hz, 4H). |
| 236 | 7.66 (d, *J* = 10.0 Hz, 1H), 6.77 (s, 1H), 6.44 (s, 1H), 3.67 (s, 3H), 3.33 (s, 2H), 2.26 (s, 3H), 2.08 (s, 3H), 1.75 (d, *J* = 1.2 Hz, 3H), 1.14 (t, *J* = 7.0 Hz, 6H). |
| 237 | 7.59 (s, 1H), 6.68 (s, 1H), 6.41 (s, 1H), 3.67 (s, 3H), 3.34 (s, 4H), 2.25 (s, 3H), 2.05 (s, 3H), 1.75 (d, *J* = 1.2 Hz, 3H), 1.57 (s, 4H), 0.86 (t, *J* = 7.4 Hz, 6H). |
| 238 | 7.71 (d, *J* = 46.4 Hz, 1H), 7.02 (d, *J* = 13.6 Hz, 1H), 6.76 (d, *J* = 0.8 Hz, 1H), 3.72 (s, 3H), 3.33 (s, 2H), 2.95 (d, *J* = 32.2 Hz, 3H), 2.11 (s, 3H), 1.75 (d, *J* = 1.1 Hz, 3H), 1.12 (q, *J*= 7.9 Hz, 3H). |
| 239 | 7.70 (s, 1H), 7.02 (s, 1H), 6.76 (s, 1H), 3.72 (s, 3H), 3.34 (s, 3H), 2.11 (s, 3H), 1.75 (s, 3H), 1.15 (d, *J* = 7.4 Hz, 7H). |
| 240 | 7.70 (s, 1H), 7.03 (s, 1H), 6.77 (d, *J* = 0.9 Hz, 1H), 3.72 (d, *J* = 0.8 Hz, 3H), 3.53 (s, 2H), 2.10 (s, 3H), 1.75 (q, *J* = 1.1 Hz, 3H), 1.62 (tq, *J* = 6.5, 3.7 Hz, 2H), 1.52 (s, 5H), 1.24 (d, J = 4.3 Hz, 1H). |
| 241 | 7.72 (s, 1H), 6.99 (s, 1H), 6.76 (s, 1H), 3.72 (s, 4H), 3.31 (s, 2H), 2.11 (d, *J* = 8.3 Hz, 3H), 1.75 (s, 3H), 1.58 (dt, *J* = 13.9, 7.1 Hz, 2H), 1.23 (s, 2H), 0.86 (q, *J* = 7.6 Hz, 7H). |
| 242 | 7.58 (s, 1H), 6.79 (s, 1H), 6.69 (s, 1H), 5.09 (s, 1H), 3.93 (q, *J* = 7.2 Hz, 2H), 2.11 (d, *J*= 8.3 Hz, 6H), 2.02 (s, 3H), 1.62 (td, *J* = 6.6, 3.2 Hz, 2H), 1.52 (q, *J* = 5.7 Hz, 5H), 1.35-1.19 (m, 6H). |
| 243 | 7.72-7.51 (m, 1H), 6.78 (s, 1H), 6.68 (s, 1H), 5.09 (s, 1H), 3.94 (q, *J=* 7.2 Hz, 2H), 2.91 (s, 3H), 2.11 (d, *J* = 4.4 Hz, 7H), 2.03 (s, 3H), 1.29 (t, *J* = 7.2 Hz, 3H), 1.12 (t, *J* = 7.1 Hz, 3H). |
| 244 | 7.58 (s, 1H), 6.79 (s, 1H), 6.68 (s, 1H), 5.09 (s, 1H), 3.94 (q, *J* = 7.2 Hz, 2H), 2.11 (d, *J* = 4.7 Hz, 6H), 2.02 (s, 3H), 1.29 (t, *J* = 7.2 Hz, 3H), 1.14 (t, *J* = 7.1 Hz, 6H). |
| 246 | 7.70 (s, 1H), 7.04 (s, 1H), 7.00 (s, 1H), 5.19 (s, 1H), 3.95 (q, *J* = 7.2 Hz, 2H), 3.54 (s, 2H), 2.13 (s, 3H), 2.04 (s, 3H), 1.62 (tt, *J* = 6.4, 3.7 Hz, 2H), 1.52 (s, 6H), 1.30 (t, *J* = 7.2 Hz, 3H). |
| 247 | 7.71 (d, *J* = 44.9 Hz, 1H), 7.04 (d, *J* = 0.9 Hz, 1H), 6.99 (d, *J* = 12.5 Hz, 1H), 5.20 (s, 1H), 3.95 (q, JJ= 7.2 Hz, 2H), 3.39 (d, *J=* 33.9 Hz, 2H), 2.95 (d, *J* = 29.8 Hz, 3H), 2.14 (s, 3H), 2.04 (s, 3H), 1.30 (t, *J=* 7.2 Hz, 3H), 1.12 (d, *J* = 7.0 Hz, 3H). |
| 248 | 7.70 (s, 1H), 7.07-7.02 (m, 1H), 7.00 (s, 1H), 5.20 (s, 1H), 3.95 (q, *J* = 7.2 Hz, 2H), 3.43 (m, *J* = 7.3 Hz, 4H), 2.14 (s, 3H), 2.04 (s, 3H), 1.30 (t, *J* = 7.2 Hz, 3H), 1.14 (t, *J* = 7.1 Hz, 6H). |
| 250 | 7.77 (s, 1H), 7.20 (s, 1H), 6.98 (s, 1H), 5.84 (s, 1H), 4.70 (hept, J*J* = 6.7 Hz, 1H), 3.49-3.35 (m, 2H), 2.93 (s, 2H), 2.16 (s, 3H), 1.44 (d, *J* = 6.7 Hz, 6H), 1.14 (t, *J* = 6.9 Hz, 4H). |
| 251 | 7.71 (s, 1H), 7.20 (s, 1H), 6.99 (s, 1H), 5.84 (s, 1H), 4.70 (hept, J = 6.6 Hz, 1H), 3.47 (d, *J* = 75.8 Hz, 4H), 2.16 (s, 3H), 1.68-1.50 (m, 6H), 1.44 (d, *J=* 6.7 Hz, 6H). |
| 252 | 7.69 (s, 1H), 7.20 (s, 1H), 6.98 (s, 1H), 5.84 (s, 1H), 4.70 (hept, *J* = 6.7 Hz, 1H), 3.45-3.29 (m, 4H), 2.16 (s, 3H), 1.44 (d, *J* = 6.7 Hz, 6H), 1.16 (t, *J* = 7.1 Hz, 6H). |
| 253 | 7.77 (s, 1H), 7.21 (s, 1H), 6.96 (s, 1H), 5.83 (s, 1H), 4.70 (hept, *J* = 6.7 Hz, 1H), 4.46-4.38 (m, 1H), 3.75-3.67 (m, 1H), 2.16 (s, 3H), 1.44 (d, *J* = 6.6 Hz, 6H), 1.27 (d, *J* = 7.3 Hz, 12H). |
| 254 | 7.62 (s, 1H), 6.89 (s, 1H), 6.73 (s, 1H), 5.68 (s, 1H), 3.95 (d, *J=* 7.3 Hz, 2H), 3.36-3.31 (m, 2H), 2.92 (s, 3H), 2.20 (dq, *J* = 13.7, 6.8 Hz, 1H), 2.13 (s, 6H), 1.13 (t, *J* = 7.1 Hz, 3H), 0.91 (d, *J* = 6.7 Hz, 6H). |
| 255 | 7.60 (s, 1H), 6.89 (s, 1H), 6.73 (s, 1H), 5.68 (s, 1H), 3.95 (d, *J* = 7.3 Hz, 2H), 3.55-3.28 (m, 6H), 2.19 (dt, *J* = 13.7, 6.9 Hz, 1H), 2.12 (s, 6H), 1.53-1.43 (m, 6H), 0.91 (d, *J* = 6.7 Hz, 6H). |
| 259 | 7.79 (s, 1H), 7.18 (s, 1H), 7.04 (s, 1H), 5.84 (s, 1H), 3.97 (d, *J* = 7.3 Hz, 2H), 3.41-3.34 (m, 2H), 2.97 (s, 3H), 2.21 (dd, *J* = 13.7, 6.9 Hz, 1H), 2.17 (s, 3H), 1.14 (t, *J* = 7.1 Hz, 3H), 0.91 (d, *J* = 6.8 Hz, 6H). |
| 260 | 7.73 (s, 1H), 7.17 (s, 1H), 7.05 (s, 1H), 5.84 (s, 1H), 3.97 (d, *J =* 7.3 Hz, 2H), 3.40-3.32 (m, 4H), 2.28-2.17 (m, 1H), 2.16 (s, 3H), 1.57-1.34 (m, 6H), 0.91 (d, *J* = 6.7 Hz, 6H). |
| 263 | 7.79 (s, 1H), 7.17 (s, 1H), 7.02 (s, 1H), 5.83 (s, 1H), 3.97 (d, *J* = 7.2 Hz, 2H), 3.52-3.32 (m, 2H), 2.23 (dt, *J* = 13.7, 6.8 Hz, 1H), 2.17 (s, 3H), 1.26 (d, *J* = 5.7 Hz, 12H), 0.92 (d, *J* = 6.6 Hz, 6H). |

Other compounds represented by General Formula I of the present application can be synthesized by referring to the methods described above.

### Formulation Example 1

In this example, a fungicide formulation was prepared using the synthesized pyrazol-5-ether compound. A suspension containing Compound 2 having a concentration of 30% was formulated according to the mass ratio shown in Table 5.

**Table 5**

| Compound 2 | 30% |
|---|---|
| ethylene glycol | 10% |
| Nonylphenol polyethylene glycol ether | 6% |
| Sodium lignosulfonate | 10% |
| Carboxymethyl cellulose | 1% |
| 37% formaldehyde aqueous solution | 0.2% |
| 75% silicone oil aqueous solution | 0.8% |
| Water | Make up to 100% |

The preparation method is: Compound 2 and other components were thoroughly mixed to obtain a 30% suspension. Diluents of desired concentrations can be obtained by diluting the resulting suspension with water.

### Bioactivity Test Example

Various kinds of pathogens were tested with the compounds of the present application. Unless otherwise specified in the examples and the present application, the preparation method of samples is as follows: 10 mg of the compound was weighed and dissolved in 1 mL DMF to prepare a 10000 ppm stock solution, and the stock solution was diluted with 0.05% Tween-80 aqueous solution to the required concentrations for activity tests.

### Test Example 1: Fungicidal activity of the compounds against wheat powdery mildew

In this example, the control effects of the prepared pyrazol-5-ether compounds against wheat powdery mildew (*Blumeria graminis (DC.) Speer)* were tested using the following method.

A whole-plant pot assay was employed for screening. The pesticide formulation was uniformly sprayed onto wheat leaves at the first true leaf stage using a laryngeal atomizer, with three replicates per treatment. The treated plant was air-dried naturally indoors. At 24 hours post-treatment, wheat powdery mildew spores (collected approximately 10 days after inoculation) were evenly dusted onto the compound-treated wheat leaves. Disease assessment was conducted 10 days after spore inoculation when symptom development stabilized in the control group. The assessment results were recorded on a 100-0 scale, where '100' indicates complete disease absence and '0' represents maximum disease severity.

The control effects of the compounds 18, 66, 68, 102, 117, 132, 143, 149, 179, 186, 225, 230, 250, 251, 254, 255, 259, and 260 against wheat powdery mildew were ≥ 80% at a concentration of 40 ppm.

The control effects of the compounds 16, 69, 175, 176, 177, 178, 185, 186, 188, 189, 207, 212, and 215 against wheat powdery mildew were > 70% at a concentration of 10 ppm.

According to the same method, the results of the activity comparison of the compounds of the present application with KC1, KC2, and KC3 are shown in Table 6.

**Table 6 Control effects of the compounds of the present application, KC1, KC2, and KC3 against wheat powdery mildew (control effect: %)**

| Compound | 40 ppm | 10 ppm |
|---|---|---|
| Compound 16 | 96.30 | 81.48 |
| Compound 132 | 88.89 | 74.07 |
| Compound 143 | 81.48 | 74.07 |
| KC1 | 22.22 | 0 |
| KC2 | 0 | 0 |
| KC3 | 59.26 | 0 |

### Test Example 2: Fungicidal activity of the compounds against soybean rust

In this example, the control effects of the prepared pyrazol-5-ether compounds against soybean rust (*Phakopsora pachyrhizi*) were tested using the following method.

A whole-plant pot assay was employed for screening. The sample of a compound to be tested was dissolved in a small amount of solvent (acetone, methanol or DMF selected based on the solubility of the sample, with solvent-to-spray solution volume ratio less than or equal to 0.05) and then diluted with 0.1% Tween-80 aqueous solution to prepare test solutions at required concentrations. Greenhouse-cultivated soybean plants at the two-leaf stage served as test hosts for soybean rust (*Phakopsora pachyrhizi*). The compounds of the present application were sprayed on the leaves at designed concentrations, and blank controls sprayed with water were also set, in triplicate. At 24 hours post-treatment, pathogen inoculation was conducted. After inoculation, the plants were maintained in controlled environment chamber for constant humidity culture (temperature: 25 °C day/20 °C night, relative humidity: 95%-99%). After 24 hours of incubation, the plants were transferred to the greenhouse. Control effect assessments were conducted on the compounds when disease symptoms fully developed in controls (typically one week). The assessment results were recorded according to the *A Manual of Assessment Keys for Plant Diseases* compiled by the American Phytopathological Society on a 100-0 scale, where '100' indicates complete disease absence and '0' represents maximum disease severity.

The control effects of the compounds 176, 212, 242, 243, 254, and 255 against soybean rust were > 90% at a concentration of 10 ppm.

The control effects of the compounds 16, 175, 199, 201, 207, and 208 against soybean rust were > 80% at a concentration of 4 ppm.

According to the above method, the control effect of the existing compound KC3 against soybean rust was 22% at a concentration of 10 ppm and 0 at a concentration of 4 ppm.

### Test Example 3: Fungicidal activity of the compounds against cucumber powdery mildew

In this example, the control effects of the prepared pyrazol-5-ether compounds against cucumber powdery mildew (*Erysiphe cichoracearum*) were tested using the following method.

A whole-plant pot assay was employed for screening. The sample of a compound to be tested was dissolved in a small amount of solvent (acetone, methanol or DMF selected based on the solubility of the sample, with solvent-to-spray solution volume ratio less than or equal to 0.05) and then diluted with 0.1% Tween-80 aqueous solution to prepare test solutions at required concentrations. Greenhouse-cultivated cucumber seedlings at the two-leaf stage served as test hosts for cucumber powdery mildew (*Erysiphe cichoracearum*). The compounds of the present application were sprayed on the leaves at designed concentrations, and lank controls sprayed with water were also set, in triplicate. At 24 hours post-treatment, pathogen inoculation was conducted. After inoculation, the plants were maintained in controlled environment chamber for constant humidity culture (temperature: 25 °C day/20 °C night, relative humidity: 95%-99%). After 24 hours of incubation, the plants were transferred to the greenhouse. Control effect assessments were conducted on the compounds when disease symptoms fully developed in controls (typically one week). The assessment results were recorded on a 100-0 scale, where '100' indicates complete disease absence and '0' represents maximum disease severity.

The control effects of the compounds 180, 185, 186, 190, 192, 198, 208, 212, 214, and 217 against cucumber powdery mildew were ≥ 90% at a concentration of 4 ppm.

The control effects of the compounds 185, 186, 190, 198, 212, and 214 against cucumber powdery mildew were > 90% at a concentration of 2.5 ppm.

According to the above method, the control effects of the existing compounds KC1 and KC2 against cucumber powdery mildew were 0 at a concentration of 2.5 ppm, and the control effect of the existing compound KC3 against cucumber powdery mildew was 22.22% at a concentration of 2.5 ppm.

Although the pyrazol-5-ether compound of the present application, the preparation method therefor and the use thereof have been illustrated through the above embodiments, the present application is not limited to the above embodiments, which means that the implementation of the present application does not necessarily depend on the above embodiments. It should be apparent to those skilled in the art that any improvements made to the present application, equivalent replacements of raw materials for preparing the products of the present application, additions of adjuvant ingredients, selections of specific manners, etc., all fall within the protection scope and the disclosure scope of the present application.

## Claims

1. A pyrazol-5-ether compound having a structure represented by Formula I: 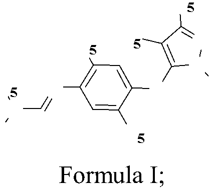 wherein
R₁ is selected from C1-C6 alkyl, C3-C6 cycloalkyl or phenyl group, which is substituted with at least one R₈;
R₂ is selected from C1-C6 haloalkyl, C3-C6 cycloalkyl, methyl, cyano or phenyl group, the phenyl group is substituted with at least one R₈;
R₃ is selected from hydrogen, halogen, cyano, C1-C6 alkyl or C1-C6 haloalkyl group;
R₄ is selected from halogen, C1-C6 alkyl, C1-C6 alkoxy, C1-C6 haloalkyl or C1-C6 haloalkoxy group;
R₅ is selected from hydrogen, halogen, C1-C6 alkyl, C1-C6 alkoxy or C1-C6 haloalkyl group;
R₆ is selected from C1-C8 alkyl, C3-C6 cycloalkyl or C1-C6 haloalkyl group, which is substituted with at least one R₈;
R₇ is selected from hydrogen or C1-C6 alkyl group; or R₆ and R₇, together with the nitrogen atom to which they are attached, form a 3- to 7-membered saturated cyclic group, the cyclic group may optionally contain an oxygen atom, a nitrogen atom or a sulfur atom, and the cyclic group may be further substituted with identical or different R₈; and
R₈ is selected from hydrogen, halogen, cyano, nitro, hydroxyl, amino, C1-C6 alkyl, C1-C6 cycloalkyl, C1-C6 haloalkyl, C1-C6 alkoxy, C1-C6 haloalkoxy, amino substituted with C1-C6 alkyl, C1-C6 alkylcarbonylamino, C1-C6 haloalkylcarbonylamino, C1-C6 alkylsulfonylamino, *N,N-di(C1-C6* alkyl)sulfonylamino, C1-C6 haloalkylsulfonylamino, heterocycloacylamino, C1-C6 alkylcarbonyl, C1-C6 alkyloxycarbonyl, cyano-substituted C1-C6 alkyl, C1-C6 alkylsulfinyl, C1-C6 alkylsulfonyl, C1-C6 haloalkylsulfinyl, C1-C6 haloalkylsulfonyl, substituted phenyl, a heterocyclic ring or heterocyclic-substituted C1-C6 alkyl group.

2. The pyrazol-5-ether compound according to claim 1, wherein
R₁ is selected from methyl, ethyl, *n*-propyl, isopropyl, *n*-butyl, isobutyl, *tert*-butyl cyclopropyl, cyclobutyl, cyclopentyl, cyclohexyl or phenyl group, which is substituted with at least one R₈;
R₂ is selected from methyl, cyclopropyl, difluoromethyl, trifluoromethyl, cyano or phenyl group, the phenyl group is substituted with at least one R₈;
R₃ is selected from hydrogen, fluorine, chlorine, bromine, iodine, cyano, methyl, difluoromethyl or trifluoromethyl group;
R₄ is selected from methyl, difluoromethyl, trifluoromethyl, methoxy, difluoromethoxy, trifluoromethoxy, fluorine, chlorine, bromine or iodine group;
R₅ is selected from hydrogen, methyl, methoxy, difluoromethyl, trifluoromethyl, fluorine, chlorine, bromine or iodine group;
R₆ is selected from methyl, ethyl, n-propyl, isopropyl, 1-methylpropyl, 1-ethylpropyl, 2-methylpropyl, 3-methylpropyl, 1,1-dimethylpropyl, 2,2-dimethylpropyl, n-butyl, tert-butyl, 3-methylbutyl, 1,1-dimethyl-3,3-dimethylbutyl, n-pentyl, 4-methyl-2-pentyl, 1-methylpentyl, 2-methylpentyl, 3-methylpentyl, 4-methylpentyl, n-hexyl, 2-ethylhexyl, cyclopropyl, cyclobutyl, cyclopentyl, cyclohexyl or cyclopropylmethyl group;
R₇ is selected from hydrogen, methyl, ethyl, n-propyl, isopropyl, n-butyl, isobutyl, tert-butyl, n-pentyl, 2-pentyl, neopentyl, isopentyl, 4-methyl-2-pentyl or n-hexyl group; or R₆ and R₇, together with the nitrogen atom to which they are attached, form tetrahydropyrrole, isoxazolidine, piperidine, azacyclooctane, piperazine or morpholine, and the tetrahydropyrrole, isoxazolidine, piperidine, azacyclooctane, piperazine or morpholine may be substituted with identical or different R₈;
R₈ is selected from hydrogen, fluorine, chlorine, bromine, iodine, cyano, nitro, hydroxyl, amino, methyl, ethyl, n-propyl, isopropyl, cyclopropyl, isobutyl, n-butyl, tert-butyl, cyclobutyl, cyanomethyl, difluoromethyl, trifluoromethyl, pentafluoroethyl, heptafluoroisopropyl, methoxy, difluoromethoxy, trifluoromethoxy, acetyl, tert-butoxycarbonyl, amino, methylamino, acetylamino, trifluoroacetylamino, methanesulfonamido, trifluoromethanesulfonamido, N,N-dimethylaminosulfonamido, morpholine-4-formamido, pyrazolyl, imidazolyl, 1,2,4-triazolyl, 1,2,3-triazolyl, 1,3,4-triazolyl, pyrazolinyl, imidazolinyl, 1,2,4-triazolidinyl, pyridyl, pyrimidinyl, pyridazinyl, pyrazinyl, oxadiazolyl, thiadiazolyl, isoxazolidinyl or isoxazolinyl group.

3. The pyrazol-5-ether compound according to claim 2, wherein
R₁ is selected from methyl, ethyl, n-propyl, isopropyl, tert-butyl, isobutyl or cyclopropyl group, which is substituted with at least one R₈;
R₂ is selected from methyl, difluoromethyl or trifluoromethyl group;
R₃ is selected from hydrogen, fluorine, chlorine, cyano or methyl group;
R₄ is selected from methyl, methoxy or chlorine group;
R₅ is selected from methyl or chlorine group;
R₆ is selected from methyl, ethyl, n-propyl or isopropyl group;
R₇ is selected from hydrogen, methyl, ethyl, n-propyl or isopropyl group; or
R₆ and R₇, together with the nitrogen atom to which they are attached, form tetrahydropyrrole, piperidine or azacyclooctane, and the tetrahydropyrrole, piperidine or azacyclooctane may be substituted with identical or different R₈; and
R₈ is selected from hydrogen, methyl or fluorine group.

4. The pyrazol-5-ether compound according to claim 3, wherein
R₁ is selected from methyl, ethyl, n-propyl, isopropyl or *tert-butyl* group;
R₂ is selected from trifluoromethyl group;
R₃ is selected from hydrogen or methyl group;
R₄ is selected from methyl or chlorine group;
R₅ is selected from methyl or chlorine group;
R₆ is selected from ethyl group;
R₇ is selected from methyl group; or
R₆ and R₇, together with the nitrogen atom to which they are attached, form piperidine, and the piperidine may be substituted with identical or different R₈; and
R₈ is selected from hydrogen or methyl group.

5. The pyrazol-5-ether compound according to any one of claims 1 to 4, wherein the pyrazol-5-ether compound is any one of the following compounds:

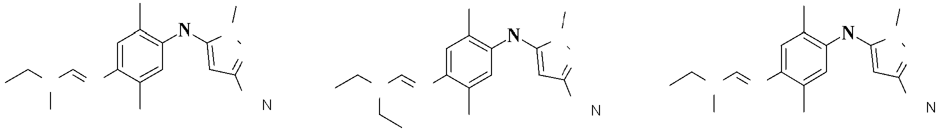

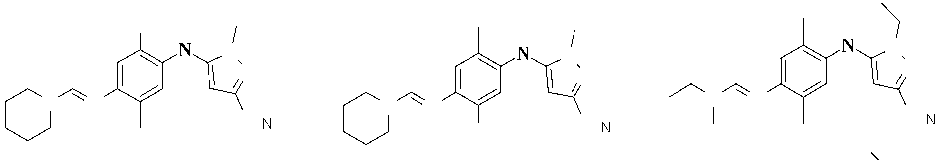

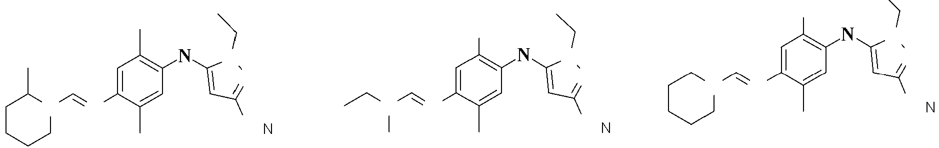

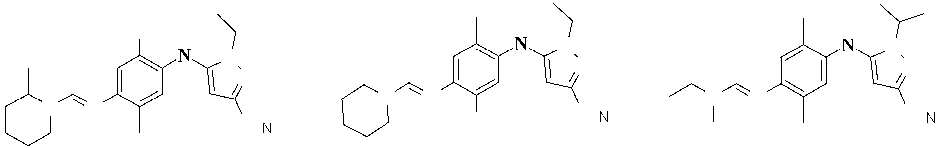

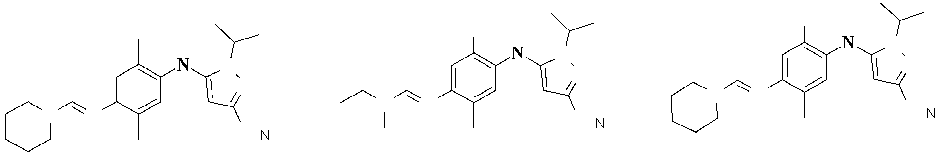

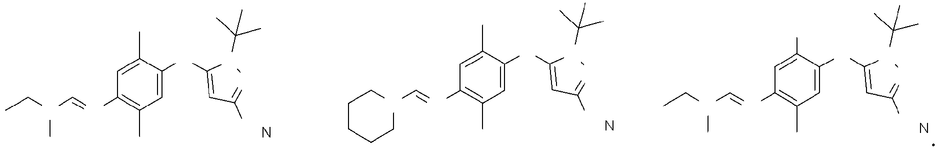

6. Use of the pyrazol-5-ether compound according to any one of claims 1 to 5 in the prevention and treatment of plant diseases.

7. A fungicide composition, comprising an active constituent and an agrochemically acceptable carrier, wherein the active constituent is the pyrazol-5-ether compound according to any one of claims 1 to 5.

8. The fungicide composition according to claim 7, wherein the active constituent has a weight percentage of 1% to 99%.

9. A method of preventing and controlling plant diseases, comprising: administering an effective dose of the fungicide composition according to claims 7 to 8 to plant diseases to be controlled or to growth media thereof.

10. The method of preventing and controlling plant diseases according to claim 9, wherein the effective dose is 10 g per hectare to 1000 g per hectare and preferably 20 g per hectare to 500 g per hectare.
